# EUROPEAN PATENT APPLICATION

(11) **EP 4 332 108 A1**
(43) Date of publication of application: **06.03.2024**
(21) Application number: 22794879.1
(22) Date of filing: 26.04.2022
(51) Int. Cl.: C07F 7/08, A61K 31/695, A61P 17/10, A61P 17/06, A61P 17/00

(54) **RETINOIC ACID RECEPTOR AGONIST, PREPARATION METHOD THEREFOR, INTERMEDIATE, PHARMACEUTICAL COMPOSITION AND USE**

(30) Priority: 27.04.2021 CN 202110459864
(71) Applicant: Jiangxi Kerui Pharmaceutical Co., Ltd., Ganzhou, Jiangxi 341000 (CN)
(72) Inventor: XU, Wenfang, Shanghai 201715 (CN); TAN, Yun, Shanghai 201715 (CN)
(74) Representative: Maiwald GmbH
(86) International application number: PCT/CN2022/089139
(87) International publication number: WO 2022/228409

(57) **Abstract**

Disclosed are a retinoic acid receptor agonist, a preparation method therefor, an intermediate, a pharmaceutical composition and a use. The structure of the compound is as shown in formula (I). The compound has relatively high agonist activity for retinoic acid receptor γ and selectivity to retinoic acid receptor γ, and has relatively low liver cell stability. It is shown that the compound has better activity in local skin, and is metabolized faster in liver cells after entering the systemic circulation. It is indicated that the drug is safer and excellent in druggability, and can potentially be used for treating diseases associated with retinoic acid receptors.

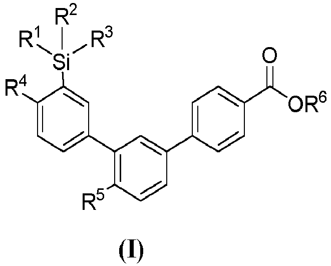

## Description

The present application claims the right of the priority of Chinese patent application 202110459864X filed on April 27, 2021. The contents of the above Chinese patent application are incorporated herein by reference in its entirety.

### TECHNICAL FIELD

The present disclosure relates to a retinoic acid receptor agonist, a preparation method therefor, an intermediate thereof, a pharmaceutical composition thereof, and a use thereof.

### BACKGROUND

Retinoic acid is a class of vitamin A metabolites that initiates and regulates gene expression through activation of nuclear receptors, which is a key regulator of cell growth, differentiation, proliferation, and programmed apoptosis, and is involved in physiopathological processes, such as embryonic development, tissue differentiation, and tumors. The physiological effects of retinoic acid are mediated by two types of receptors, the retinoic acid receptor (RAR) and the retinoid X receptor (RXR). Among them, the retinoic acid receptor (RAR) belongs to the nuclear receptor superfamily, including α, β, and γ. RARβ is subdivided into β1, β2, β3, β4, etc. The signaling of retinoic acid receptor ultimately functions through gene transcriptional regulation, and a complete signaling pathway of retinoic acid consists of the ligand retinoic acid, receptor dimerization, the retinoic acid response element (RARE), and coregulators.

The distribution of retinoic acid receptor (RAR) is temporally and spatially specific during cell development, and different retinoic acid receptor pathways have different functions for cell development. In general, RARα is most widely expressed in cells and is present in most tissues; RARβ is highly expressed in the central nervous system; and RARγ is only expressed in skin, epithelial, and cartilage tissues. In humans and adult rats, expression products of RARγ are highly restricted to the skin, with low expression in other organs. Studies have shown that RARγ selective agonists have efficacy in the treatment of conditions, such as facial acne and psoriasis.

For example, Trifarotene (trade name: Aklief) is a RARγ selective agonist developed by Galderma Research and Development, which was approved by the US FDA in 2019 for the treatment of facial acne in adolescents aged 9 years and older (Lesley J. Scott, Drugs 2019, 79, 1905-1909). Thoreau et al. reported in the literature (Bioorganic & Medicinal Chemistry Letters 2018, 28, 1736-1741) that compound 10d is also a RARγ selective agonist:

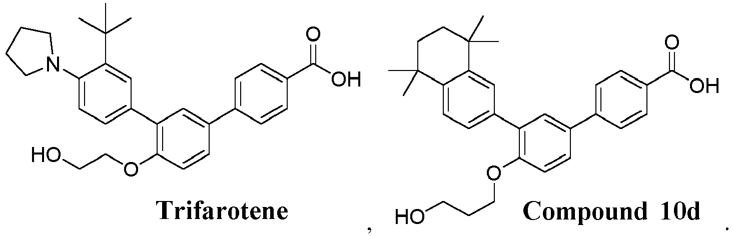

### CONTENT OF THE PRESENT INVENTION

The present disclosure is to provide a retinoic acid receptor agonist, a preparation method therefor, an intermediate thereof, a pharmaceutical composition thereof, and a use thereof.

On the one hand, the present disclosure provides a compound of formula (I): 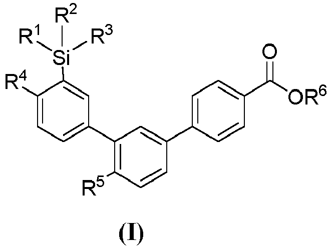
or a tautomer thereof, a stereoisomer thereof, an isotope derivative thereof, or a pharmaceutically acceptable salt thereof; wherein
R¹, R², and R³ are each independently C₁₋₆ alkyl; or, any two of R¹, R², and R³, together with the Si atom to which they are attached, form a 4- to 12-membered heterocycloalkyl ring, wherein the 4- to 12-membered heterocycloalkyl ring comprises 1, 2, 3, or 4 heteroatoms independently selected from Si, N, O, and S;
R⁴ is C₁₋₆ alkyl, -NR^{4a}R^{4b}, or -SiR^{4c}R^{4d}R^{4e}; or, R⁴ and any one of R¹, R², and R³, together with the atom to which they are attached, render 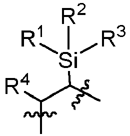 form 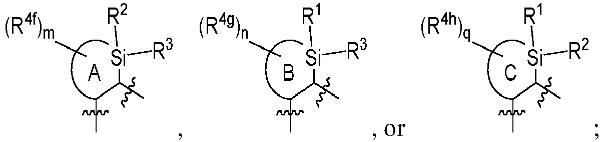
R^{4a} and R^{4b} are each independently C₁₋₆ alkyl; or, R^{4a} and R^{4b}, together with the N atom to which they are attached, form a 4- to 6-membered heterocycloalkyl ring, and the 4- to 6-membered heterocycloalkyl ring comprises 1, 2, or 3 N atoms; R^{4c}, R^{4d}, and R^{4e} are each independently C₁₋₆ alkyl;
ring A, ring B, and ring C are each independently a 4- to 12-membered heterocyclic ring, and the 4- to 12-membered heterocyclic ring comprises 1, 2, 3, or 4 heteroatoms independently selected from Si, N, O, and S; R^{4f}, R^{4g}, or R^{4h} are each independently C₁₋₆ alkyl; m, n, and q are each independently 0, 1, 2, or 3; R⁵ is C₁₋₆ alkoxy or C₁₋₆ alkoxy substituted by one or more than one R^{5a};
each R^{5a} is independently halogen or hydroxyl;
R⁶ is H or C₁₋₆ alkyl.

In some embodiments, in the definition of R¹, R², and R³, each C₁₋₆ alkyl may be independently C₁₋₄ alkyl, for example, methyl, ethyl, *n*-propyl, isopropyl, *n*-butyl, isobutyl, or *tert*-butyl, for another example, methyl.

In some embodiments, in the definition of R¹, R², and R³, when any two of R¹, R², and R³, together with the Si atom to which they are attached, form a 4- to 12-membered heterocycloalkyl ring, each 4- to 12-membered heterocycloalkyl ring may be independently a 5- to 10-membered heterocycloalkyl ring, for example, a 5- to 6-membered heterocycloalkyl ring. Each 4- to 12-membered heterocycloalkyl ring may comprise independently 2 Si atoms and 0, 1, or 2 heteroatoms independently selected from N, O, and S, for example, 2 Si atoms.

In some embodiments, in the definition of R⁴, each C₁₋₆ alkyl is independently C₁₋₄ alkyl, for example, methyl, ethyl, *n*-propyl, isopropyl, *n*-butyl, isobutyl, or *tert*-butyl*.*

In some embodiments, in the definition of R^{4a} and R^{4b}, each C₁₋₆ alkyl may be independently C₁₋₄ alkyl, for example, methyl, ethyl, *n*-propyl, isopropyl, *n*-butyl, isobutyl, or *tert*-butyl, for another example, ethyl.

In some embodiments, in the definition of R^{4a} and R^{4b}, when R^{4a} and R^{4b}, together with the N atom to which they are attached, form a 4- to 6-membered heterocycloalkyl ring, the 4- to 6-membered heterocycloalkyl ring may be a 5-membered heterocycloalkyl ring; the 4- to 6-membered heterocycloalkyl ring may comprise 1 N atom. The 4- to 6-membered heterocycloalkyl ring may be

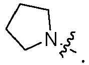

In some embodiments, in the definition of R⁴, the -NR^{4a}R^{4b} may be 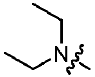 or

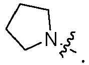

In some embodiments, in the definition of R^{4c}, R^{4d}, and R^{4e}, each C₁₋₆ alkyl may be independently C₁₋₄ alkyl, for example, methyl, ethyl, *n*-propyl, isopropyl, *n*-butyl, isobutyl, or *tert-butyl.*

In some embodiments, in the definition of R^{4f}, R^{4g}, and R^{4h}, each C₁₋₆ alkyl may be independently C₁₋₄ alkyl, for example, methyl, ethyl, *n*-propyl, isopropyl, *n*-butyl, isobutyl, or *tert*-butyl, for another example, methyl.

In some embodiments, in the definition of ring A, ring B, and ring C, each 4- to 12-membered heterocyclic ring may be independently a 5- to 10-membered heterocyclic ring, for example, a 5- to 6-membered heterocyclic ring, for another example, a 6-membered heterocyclic ring. Each 4- to 12-membered heterocyclic ring may comprise independently 2 Si atoms and 0, 1, or 2 heteroatoms independently selected from N, O, and S, for example, 2 Si atoms. The 4- to 12-membered heterocyclic ring may be a 6-membered heterocyclic ring with 2 Si atoms.

In some embodiments, in the definition of R⁵, the C₁₋₆ alkoxy may be C₁₋₄ alkoxy, for example, methoxy, ethoxy, *n*-propoxy, isopropoxy, *n*-butoxy, isobutoxy, or *tert*-butoxy, for another example, ethoxy, *n*-propoxy, or *n*-butoxy.

In some embodiments, in the definition of R^{5a}, the halogen may be fluorine, chlorine, bromine, or iodine.

In some embodiments, in the definition of R⁵, the C₁₋₆ alkoxy substituted by one or more than one R^{5a} may be C₁₋₆ alkoxy substituted by one R^{5a}, for example, C₁₋₆ alkoxy substituted by one hydroxyl group, for another example, -OCH₂CH₂OH, -OCH₂CH₂CH₂OH, or -OCH₂CH₂CH₂CH₂OH.

In some embodiments, in the definition of R⁶, the C₁₋₆ alkyl may be C₁₋₄ alkyl, for example, methyl, ethyl, *n*-propyl, isopropyl, *n*-butyl, isobutyl, or *tert-butyl.*

In some embodiments, R¹, R², and R³ may be each independently C₁₋₆ alkyl, for example, methyl.

In some embodiments, any two or all of R¹, R², and R³ may be each independently C₁₋₆ alkyl, for example, methyl;

In some embodiments, R⁴ may be -NR^{4a}R^{4b}, for example,

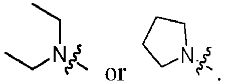

In some embodiments, R^{4a} may be ethyl.

In some embodiments, R^{4b} may be ethyl.

In some embodiments, R^{4a} and R^{4b} may be ethyl.

In some embodiments, each R^{4f} may be independently methyl.

In some embodiments, each R^{4g} may be independently methyl.

In some embodiments, each R^{4h} may be independently methyl.

In some embodiments, m may be 2.

In some embodiments, n may be 2.

In some embodiments, q may be 2.

In some embodiments, 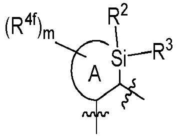 may be 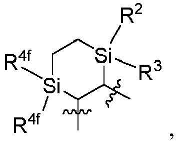 for example,

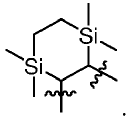

In some embodiments, 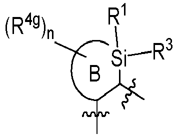 may be 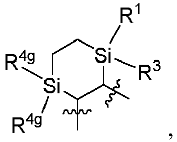 for example,

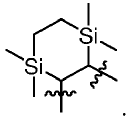

In some embodiments, 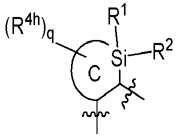 may be 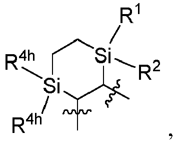 for example,

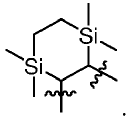

In some embodiments, R⁵ may be C₁₋₆ alkoxy substituted by one or more than one R^{5a}, for example, C₁₋₆ alkoxy substituted by one R^{5a}, for another example, -OCHzCHzOH, - OCH₂CH₂CH₂OH, or -OCH₂CH₂CH₂CH₂OH.

In some embodiments, R^{5a} may be hydroxyl.

In some embodiments, R⁶ may be H.

In some embodiments, R^{5a} is hydroxyl, and R⁶ is H.

In some embodiments, R¹, R², and R³ are each independently C₁₋₆ alkyl;
R⁴ is -NR^{4a}R^{4b};
R^{4a} and R^{4b} are each independently C₁₋₆ alkyl; or, R^{4a} and R^{4b}, together with the N atom to which they are attached, form a 4- to 6-membered heterocycloalkyl ring, and the 4- to 6-membered heterocycloalkyl ring comprises 1, 2 or 3 N atoms;
R⁵ is C₁₋₆ alkoxy substituted by one or more than one R^{5a};
each R^{5a} is independently halogen or hydroxyl;
R⁶ is H or C₁₋₆ alkyl.

In some embodiments, two of R¹, R², and R³ are each independently C₁₋₆ alkyl;
R⁴ and any one of R¹, R², and R³, together with the atom to which they are attached, render 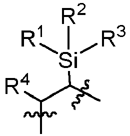 form 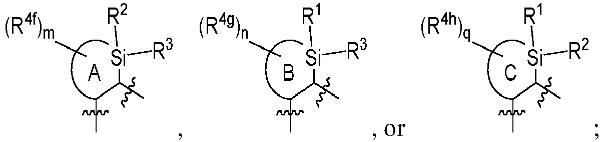
wherein ring A, ring B, and ring C are each independently a 4- to 12-membered heterocyclic ring, and the 4- to 12-membered heterocyclic ring comprises 1, 2, 3, or 4 heteroatoms independently selected from Si, N, O, and S;
R^{4f}, R^{4g}, or R^{4h} are each independently C₁₋₆ alkyl;
m, n, and q are each independently 0, 1, 2, or 3;
R⁵ is C₁₋₆ alkoxy substituted by one or more than one R^{5a};
each R^{5a} is independently halogen or hydroxyl;
R⁶ is H or C₁₋₆ alkyl.

In some embodiments, the compound of formula (I) has a structure of formula (I-a): 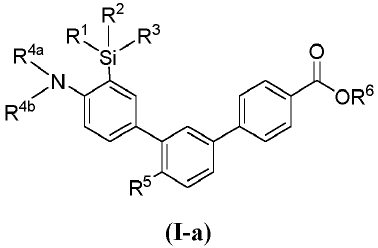 wherein R¹, R², R³, R^{4a}, R^{4b}, R⁵, and R⁶ are as defined in any embodiment of the present disclosure.

In some embodiments, the compound of formula (I) has a structure of formula (I-b): 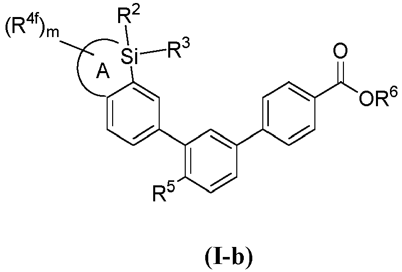 wherein ring A, m, R^{4f}, R², R³, R⁵, and R⁶ are as defined in any embodiment of the present disclosure.

In some embodiments, the compound of formula (I) has a structure of formula (I-c): 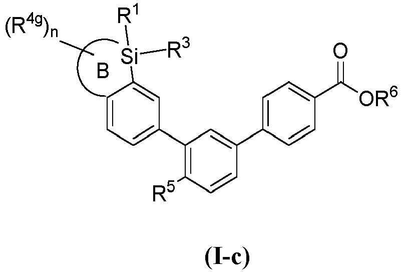 wherein ring B, n, R^{4g}, R¹, R³, R⁵, and R⁶ are as defined in any embodiment of the present disclosure.

In some embodiments, the compound of formula (I) has a structure of formula (I-d): 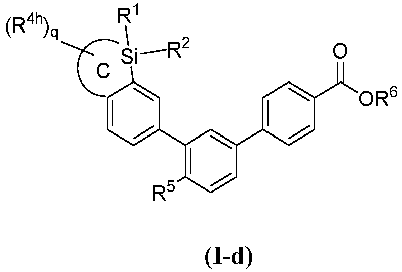 wherein ring C, q, R^{4h}, R¹, R², R⁵, and R⁶ are as defined in any embodiment of the present disclosure.

In some embodiments, the compound of formula (I) has a structure of formula (I-b-1): 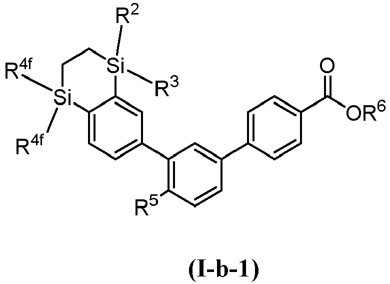 wherein R^{4f}, R², R³, R⁵, and R⁶ are as defined in any embodiment of the present disclosure.

In some embodiments, the compound of formula (I) has a structure of formula (I-c-1): 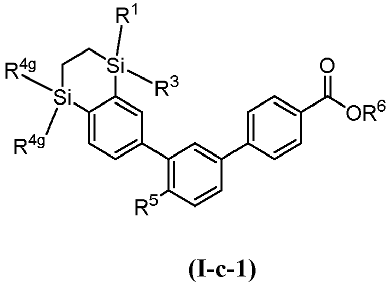 wherein R^{4g}, R¹, R³, R⁵, and R⁶ are as defined in any embodiment of the present disclosure.

In some embodiments, the compound of formula (I) has a structure of formula (I-d-1): 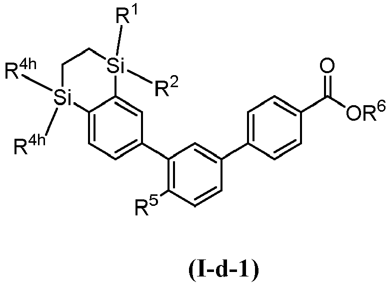 wherein R^{4h}, R¹, R², R⁵, and R⁶ are as defined in any embodiment of the present disclosure.

In some embodiments, the compound of formula (I) may be:

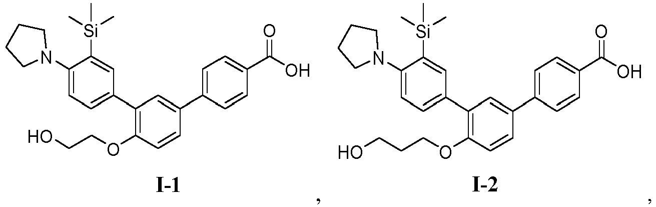

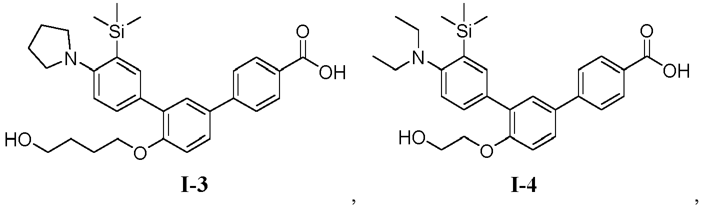

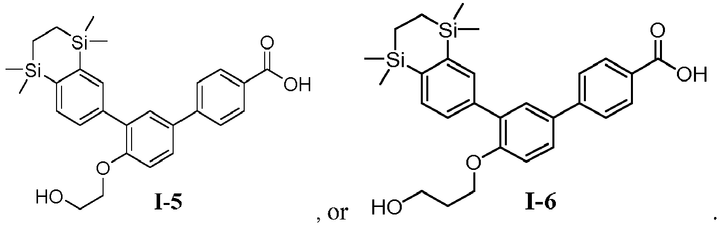

On the other hand, the present disclosure also provides a preparation method for the compound of formula (I), which is any one of the following methods:
method (A) comprises the following steps: in a solvent, reacting a compound of formula (II-a) as shown below in the presence of a base to obtain the compound of formula (I); wherein R⁵ contains a hydroxyl group, R^{5a'} is a group in which the hydroxyl group in R⁵ is protected by a hydroxyl protecting group (for example, an acetyl group), R⁶ is H, and 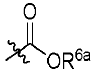 is a group in which 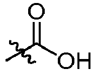 is protected by a carboxyl protecting group (for example, 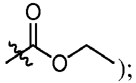 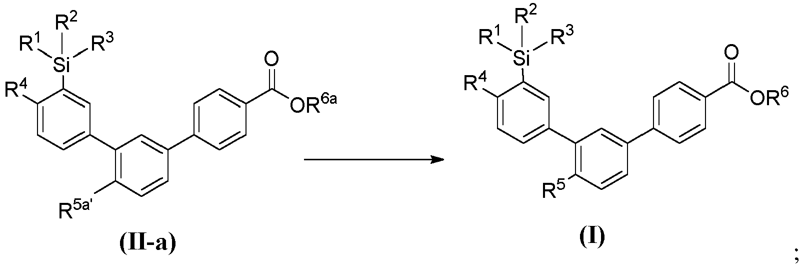
method (B) comprises the following steps: in a solvent, reacting a compound of formula (II-b) as shown below in the presence of a base to obtain the compound of formula (I); wherein R⁵ does not contain a hydroxyl group, R⁶ is H, R^{5b} and R⁵ are the same, and 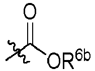 is a group in which 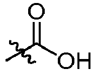 is protected by a carboxyl protecting group (for example, 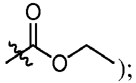 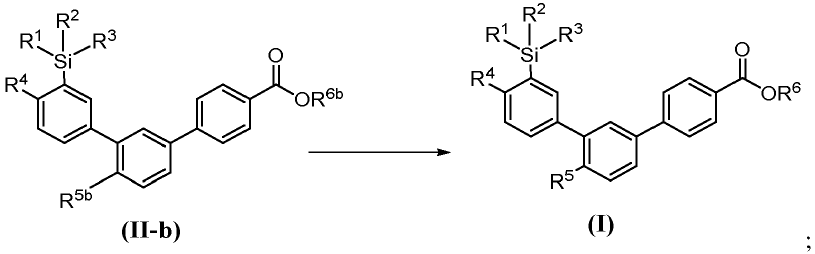
method (C) comprises the following steps: in a solvent, reacting a compound of formula (II-c) as shown below in the presence of a base to obtain the compound of formula (I); wherein R⁵ contains a hydroxyl group, R⁶ is not H, R^{5c} is a group in which the hydroxyl group in R⁵ is protected by a hydroxyl protecting group (for example, an acetyl group), and R^{6c} and R⁶ are the same; 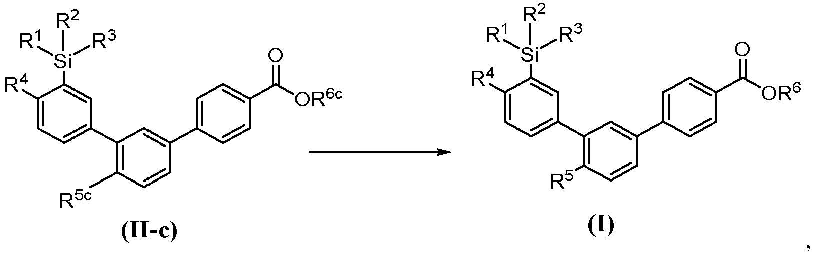
each of the rest groups is as defined above.

In the preparation method for the compound of formula (I), the reaction conditions and reagents of method (A), method (B), or method (C) may be the conventional conditions and reagents of this type of reaction in the art, and the following conditions and reagents are preferred for the present disclosure.

In some embodiments, the solvent in the method (A), method (B), or method (C) may be an ether solvent, for example, tetrahydrofuran. The base may be an inorganic base (for example, sodium hydroxide); the base can be used in the form of an aqueous solution, and the concentration of the aqueous solution of the base may be 1 M to 2 M. The temperature of the reaction may be 20°C to 50°C, for example, 40°C.

In some embodiments, the process of the reaction in the method (A), method (B), or method (C) can be monitored using conventional test methods in the art (for example, TLC, GC, HPLC, or NMR), and those skilled in the art can determine when to terminate the reaction based on the results of the monitoring (including the degree of conversion of raw materials, the generation of impurities, etc.) to obtain a better result of the reaction, and the reaction time of the reaction may be 2 hours.

In some embodiments, the preparation method for the compound of formula (II-a) comprises the following steps: in a solvent, performing a coupling reaction as shown below between a compound of formula (III-a) and a compound of formula (IV) (e.g., the condition can be in the presence of a base and a catalyst) to obtain the compound of formula (II-a); 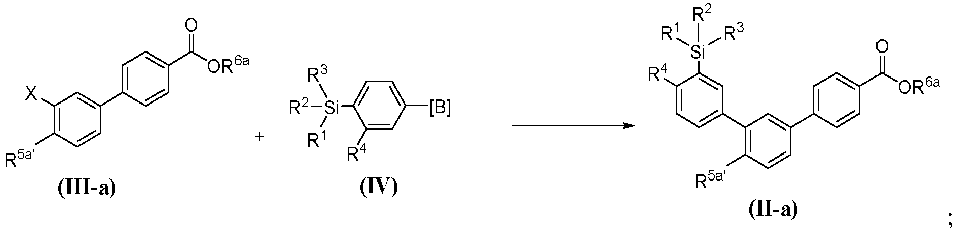
the preparation method for the compound of formula (II-b) comprises the following steps: in a solvent, performing a coupling reaction as shown below between a compound of formula (III-b) and the compound of formula (IV) (e.g., the condition can be in the presence of a base and a catalyst) to obtain the compound of formula (II-b); 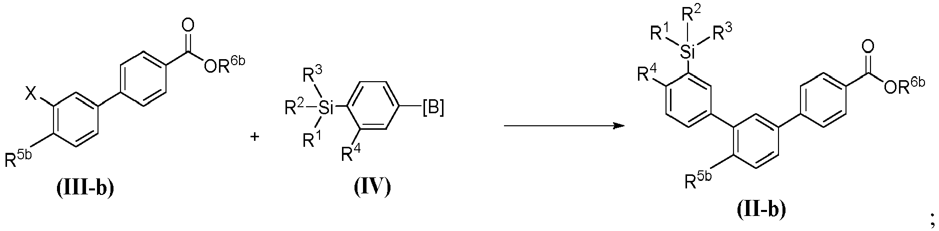
the preparation method for the compound of formula (II-c) comprises the following steps: in a solvent, performing a coupling reaction as shown below between a compound of formula (III-c) and the compound of formula (IV) (e.g., the condition can be in the presence of a base and a catalyst) to obtain the compound of formula (II-c); 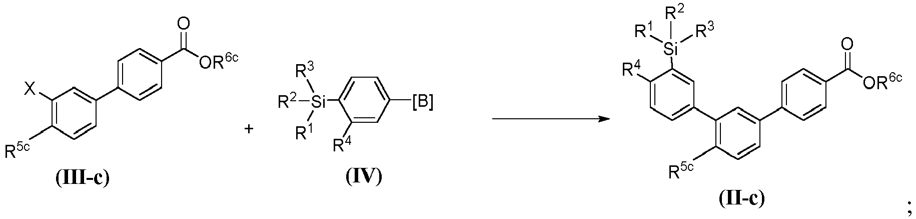
wherein X is Br or I (for example, Br), [B] is a boric acid or borate ester (for example, 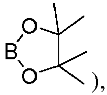 and other groups are as defined above.

In some embodiments, the preparation method for the compound of formula (I) may further comprise a preparation method for the compound of formula (II-a), (II-b), or (II-c).

In the preparation method for the compound of formula (II-a), (II-b), or (II-c), the reaction conditions and reagents of the coupling reaction may be conventional conditions and reagents of this type of reaction in the art, and the following conditions and reagents are preferred for the present disclosure.

In some embodiments, in the preparation method for the compound of formula (II-a), (II-b), or (II-c), the solvent may be a mixed solvent of an ether solvent (for example, dioxane) and water; in the mixed solvent, the volume ratio of the ether solvent and water may be 4:1. The base may be an inorganic base (for example, sodium bicarbonate). The catalyst is a palladium catalyst (for example, Pd(dppf)Cl₂). The temperature of the coupling reaction may be 90°C to 130°C, for example, 120°C. The coupling reaction can be carried out under microwave irradiation and/or in an inert (for example, nitrogen) atmosphere.

In some embodiments, in the preparation method for the compound of formula (II-a), (II-b), or (II-c), the process of the coupling reaction can be monitored using conventional test methods in the art (for example, TLC, GC, HPLC, or NMR), and those skilled in the art can determine when to terminate the reaction based on the results of the monitoring (including the degree of conversion of raw materials, the generation of impurities, etc.) to obtain a better result of the reaction, and the reaction time of the reaction may be 1 hour.

In some embodiments, a preparation method for the compound of formula (IV):
when R⁴ in the compound of formula (IV) is C₁₋₆ alkyl, -NR^{4a}R^{4b}, or -SiR^{4c}R^{4d}R^{4e}, and [B] is 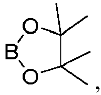 the preparation method comprises the following steps: in a solvent, performing a coupling reaction as shown below between a compound of formula (V) and bis(pinacolato)diboron (e.g., the condition can be in the presence of a base and a catalyst) to obtain the compound of formula (IV); 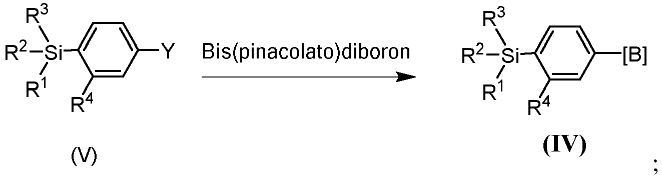
wherein Y is Br or I (for example, Br);
when the compound of formula (IV) is a compound of formula (IV-a-1), (IV-b-1), or (IV-c-1), the preparation method comprises the following steps: in a solvent, performing a cyclization reaction as shown below between a compound of formula (VI-a), (VI-b), or (VI-c) and 2-ethynyl-4,4,5,5-tetramethyl-[1,3,2]dioxaborolane (e.g., in the presence of a cobalt catalyst (for example, cobalt iodide) and zinc) to obtain the compound of formula (IV-a-1), (IV-b-1), or (IV-c-1), respectively;

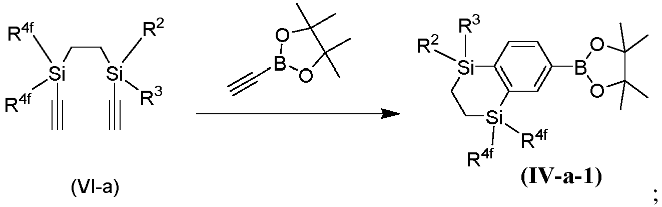

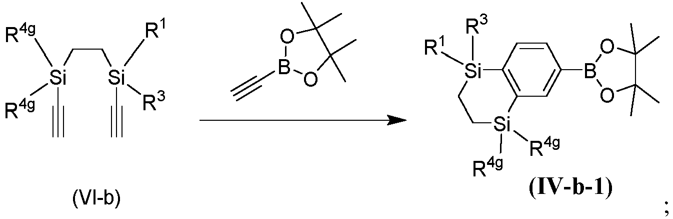

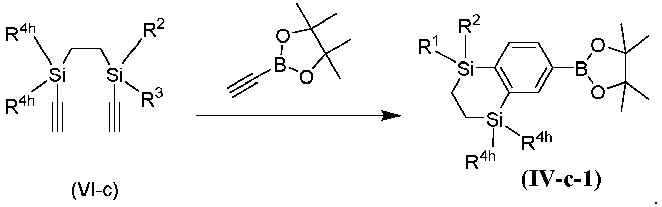

Among them, each group is as defined above.

In some embodiments, the preparation method for the compound of formula (I), (II-a), (II-b), or (II-c) may further comprise a preparation method of the compound of formula (IV).

In the preparation method of the compound of formula (IV), the reaction conditions and reagents of the reaction may be conventional conditions and reagents of this type of reaction in the art, and the following conditions and reagents are preferred for the present disclosure.

In some embodiments, in the preparation method of the compound of formula (IV), the solvent of the coupling reaction may be an ether solvent (for example, dioxane). The base of the coupling reaction may be an inorganic base (for example, potassium acetate). The catalyst of the coupling reaction is a palladium catalyst (for example, Pd(dppf)Cl₂). The temperature of the coupling reaction may be 90°C to 130°C, for example, 120°C. The coupling reaction can be carried out under microwave irradiation and/or in an inert (for example, nitrogen) atmosphere. The solvent of the cyclization reaction may be acetonitrile (anhydrous acetonitrile). The temperature of the cyclization reaction may be 20°C to 60°C, for example, 50°C. The cyclization reaction can be carried out in an inert (for example, argon) atmosphere.

In some embodiments, in the preparation method for the compound of formula (IV), the process of the reaction can be monitored using conventional test methods in the art (for example, TLC, GC, HPLC, or NMR), and those skilled in the art can determine when to terminate the reaction based on the results of the monitoring (including the degree of conversion of raw materials, the generation of impurities, etc.) to obtain a better result of the reaction. The reaction time of the coupling reaction may be 1 hour. The reaction time of the cyclization reaction may be 1 hour.

On the other hand, the present disclosure also provides a compound of formula (V), (IV), (II-a), (II-b), or (II-c): 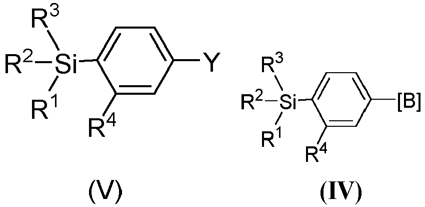 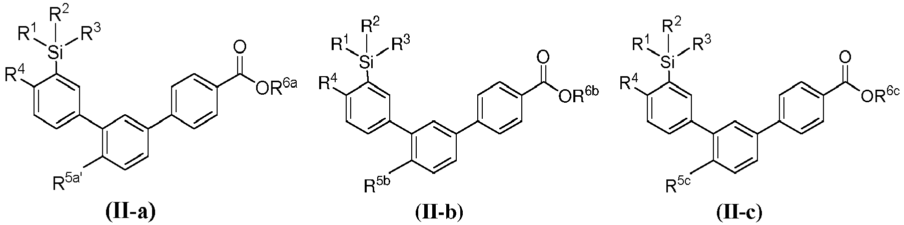 wherein R¹, R², R³, R⁴, R^{5a'}, R^{5b}, R^{5c}, R^{6a}, R^{6b}, R^{6c}, [B], and Y are as defined in any embodiment of the present disclosure.

In some embodiments, the compound of formula (V) may be: 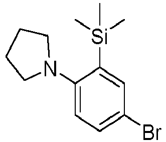 or

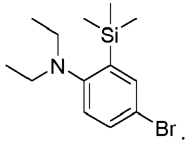

In some embodiments, the compound of formula (IV) may be:

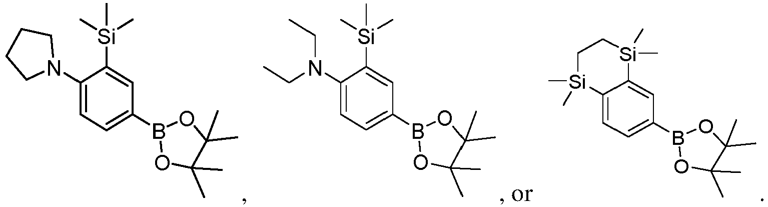

In some embodiments, the compound of formula (II-a) may be:

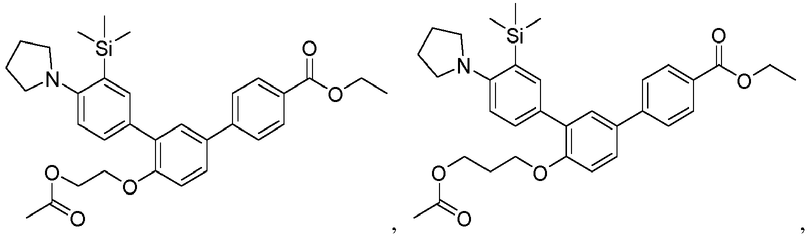

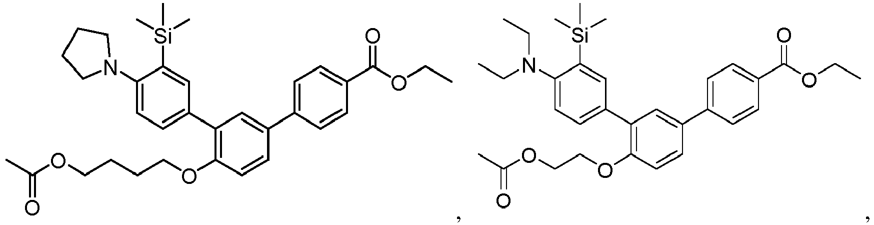

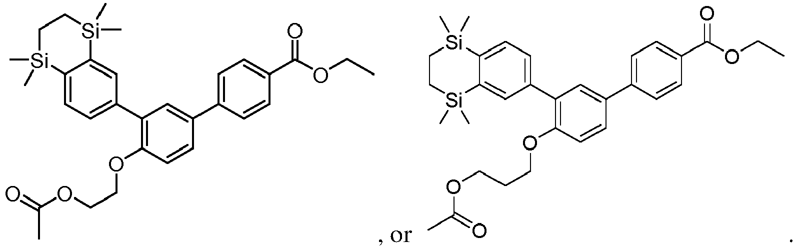

On the other hand, the present disclosure also provides a pharmaceutical composition, comprising (i) the compound of formula (I), the tautomer thereof, the stereoisomer thereof, the isotope derivative thereof, or the pharmaceutically acceptable salt thereof, and (ii) a pharmaceutically acceptable carrier.

On the other hand, the present disclosure also provides a use of the compound of formula (I), the tautomer thereof, the stereoisomer thereof, the isotope derivative thereof, or the pharmaceutically acceptable salt thereof as a medicament.

On the other hand, the present disclosure also provides a use of the compound of formula (I), the tautomer thereof, the stereoisomer thereof, the isotope derivative thereof, or the pharmaceutically acceptable salt thereof, or the pharmaceutical composition as a retinoic acid receptor (e.g., retinoic acid receptor γ) agonist.

The present disclosure also provides a use of the compound of formula (I), the tautomer thereof, the stereoisomer thereof, the isotope derivative thereof, or the pharmaceutically acceptable salt thereof, or the pharmaceutical composition in the manufacture of a medicament for the treatment or prevention of a disease associated with retinoic acid receptor (e.g., retinoic acid receptor γ).

In the present disclosure, the disease associated with retinoic acid receptor (e.g., retinoic acid receptor γ) may be acne (including common acne, rosacea, nodulocystic acne, acne conglobata, and secondary acne caused by sun exposure or medication therapy), pimples, psoriasis, ichthyosis, keratosis, hyperpigmentation, dry eye syndrome, etc.

On the other hand, the present disclosure also provides a method for the treatment of a disease associated with retinoic acid receptor (e.g., retinoic acid receptor γ), comprising administering to a subject in need of the treatment the compound of formula (I) (preferably, administering to a subject in need of the treatment a therapeutically effective amount of the compound of formula (I)), the tautomer thereof, the stereoisomer thereof, the isotope derivative thereof, or the pharmaceutically acceptable salt thereof, or the pharmaceutical composition.

The positive and progressive effect of the present disclosure is to provide a retinoic acid receptor agonist, a preparation method therefor, an intermediate thereof, a pharmaceutical composition thereof, and a use thereof. The compound has relatively high agonist activity for retinoic acid receptor γ and selectivity to retinoic acid receptor γ, and has relatively low hepatocyte stability. It is shown that the compound has better activity in local skin, and is metabolized faster in hepatocytes after entering the systemic circulation. It is indicated that the drug is safer and excellent in druggability, and can potentially be used for treating diseases associated with retinoic acid receptor (e.g., retinoic acid receptor γ).

Unless otherwise specified, the terms used in the present disclosure have the following definitions, and the definitions of terms not involved below are as commonly understood by those skilled in the art.

The term "tautomer" refers to a functional group isomer resulting from the rapid movement of an atom in a molecule between two positions. For example, acetone and 1-propen-2-ol can be transformed into each other by rapid movement of a hydrogen atom on the oxygen and α-carbon.

The term "stereoisomer" refers to isomers in which the atoms or groups of atoms in the molecule are connected in the same order but with different spatial arrangements, for example, *cis-trans* isomers (for example, *Z*-isomers, *E*-isomers), optical isomers (for example, enantiomers, diastereomers), atropisomers, etc. These stereoisomers can be separated, purified, and enriched by asymmetric synthesis methods or chiral separation methods (including, but not limited to, thin-layer chromatography, rotary chromatography, column chromatography, gas chromatography, high-pressure liquid chromatography), or these stereoisomers can be obtained by chiral resolution through bonding (for example, chemical bonding) or salt formation (for example, physical bonding) with other chiral compounds. Optical isomers include enantiomers and diastereomers. All these isomers and their mixtures are included within the scope of the present disclosure.

The term "isotope derivative" refers to a compound in which one or more than one atom is substituted by one or more than one atom with a specific atomic mass or mass number. Examples of isotopes that can be incorporated into a compound include, but are not limited to, isotopes of hydrogen, carbon, nitrogen, oxygen, fluorine, sulfur, and chlorine (for example, ²H, ³H, ¹³C, ¹⁴C, ¹⁵N, ¹⁸O, ¹⁷O, ¹⁸F, ³⁵S, and ³⁶Cl). Isotope compounds can typically be prepared by replacing non-isotope-labeled reagents with isotope-labeled reagents according to the methods described herein. Typical examples of isotope derivatives include a deuterated compound.

The term "pharmaceutically acceptable salt" refers to a salt prepared by a compound with a relatively non-toxic, pharmaceutically acceptable acid or base. When the compound contains a relatively acidic functional group, a base addition salt can be obtained by bringing the neutral form of the compound into contact with a sufficient amount of a pharmaceutically acceptable base in a pure solution or a suitable inert solvent. When the compound of the present disclosure contains a relatively basic functional group, an acid addition salt can be obtained by bringing the neutral form of the compound into contact with a sufficient amount of a pharmaceutically acceptable acid in a pure solution or a suitable inert solvent. When the compound contains a relatively acidic functional group and a relatively basic functional group, it can be converted into a base addition salt or an acid addition salt.

The term "halogen" means fluorine, chlorine, bromine, or iodine.

The term "hydroxyl" means an -OH group.

The term "alkyl" refers to a saturated linear or branched monovalent hydrocarbon group with a certain number of carbon atoms. C₁₋₆ alkyl refers to an alkyl group with 1 to 6 (for example, 1, 2, 3, 4, 5, or 6) carbon atoms, including C₁ alkyl, C₂ alkyl, C₃ alkyl, C₄ alkyl, C₅ alkyl, and C₆ alkyl, for example, methyl, ethyl, *n*-propyl, isopropyl, *n*-butyl, isobutyl, *tert-*butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc.

The term "alkoxy" refers to the group -O-R^{X}, wherein R^{X} is alkyl as defined above.

The term "x- to y-membered" in the cyclic group described herein means that the number of atoms in the ring is x to y. For example, cyclopropyl is 3-membered, tetrahydropyrrolyl is 5-membered, and piperidinyl is 6-membered.

The term "substituted" or "substituent" means that one or more than one hydrogen atom is replaced by a specified group. When the substitution position is not specified, the substitution can be at any position, but is only allowed if it forms a stable or chemically available chemical.

The term "protecting group" includes, but is not limited to, "hydroxyl protecting group" or "carboxyl protecting group", and refers to a protecting group suitable for preventing side reactions of the group. The term "hydroxyl protecting group" refers to a protecting group suitable for preventing the side reaction on hydroxyl. Representative hydroxyl protecting groups include, but are not limited to: alkyl, for example, methyl, ethyl, and *tert*-butyl; acyl, for example, alkanoyl (e.g., acetyl); arylmethyl, for example, benzyl (Bn), *p*-methoxybenzyl (PMB), 9-fluorenylmethyl (Fm), and diphenylmethyl (benzhydryl, DPM); silyl, for example, trimethylsilyl (TMS) and *tert*-butyl dimethylsilyl (TBS).

The term "treatment" refers to therapeutic therapy. When referring to a specific condition, treatment refers to: (1) alleviating the disease or one or more than one biological manifestation of the condition, (2) interfering with (a) one or more than one point in the biological cascade that leads to or causes the condition or (b) one or more than one biological manifestation of the condition, (3) ameliorating one or more than one symptom, effect, or side effect associated with the condition, or one or more than one symptom, effect, or side effect associated with the condition or the treatment thereof, or (4) slowing the progression of the condition or one or more than one biological manifestation of the condition.

The term "therapeutically effective amount" refers to the amount of a compound that is sufficient to effectively treat or prevent the disease or condition described herein when administered to a patient. The "therapeutically effective amount" will vary depending on the compound, the condition and its severity, and the age of the patient to be treated, but it can be adjusted as necessary by those skilled in the art. Doses beyond this range can also be used depending on the dosage form and the severity of the disease.

The pharmaceutical composition can be formulated into various types of unit dosage forms according to the therapeutic purpose.

The compounds of the present disclosure can be administered clinically in a conventional manner.

The term "subject" refers to any animal, preferably a mammal, and most preferably a human, that is to be or has been administered a compound or composition. The term "mammal" includes any mammal. Examples of mammals include, but are not limited to, cattle, horses, sheep, pigs, cats, dogs, mice, rats, rabbits, guinea pigs, monkeys, humans, etc., most preferably humans.

The following abbreviations are used herein: K₂CO₃ represents potassium carbonate; CH₃CN or MeCN represents acetonitrile; *n*-BuLi represents n-butyllithium; TMSCl represents chlorotrimethylsilane; TMS represents trimethylsilyl; B₂Pin₂ represents bis(pinacolato)diboron; KOAc represents potassium acetate; Pd(dppf)Cl₂ represents [1,1'-bis(diphenylphosphino)ferrocene]dichloropalladium(II); MW represents microwave; NBS represents *N*-bromosuccinimide; DCM represents dichloromethane; NaH represents sodium hydride; DMF represents *N,N*-dimethylformamide; NaHCO₃ represents sodium bicarbonate; KI represents potassium iodide; NaOH represents sodium hydroxide; PE represents petroleum ether; EA represents ethyl acetate; THF represents tetrahydrofuran; TLC represents thin-layer chromatography; HPLC represents high-pressure liquid chromatography; LCMS represents liquid chromatography mass spectrometry.

All patents and publications referred to herein are incorporated herein by reference in their entirety.

On the basis of common knowledge in the art, the above-mentioned preferred conditions can be combined arbitrarily to obtain preferred examples of the present disclosure.

The reagents and raw materials used in the present disclosure are all commercially available or can be prepared according to existing methods.

### DETAILED DESCRIPTION OF THE PREFERRED EMBODIMENT

The present disclosure is further illustrated below by means of examples, but the present disclosure is not limited to the scope of the examples. The experimental methods that haven't illustrated specific conditions in the following examples are selected according to conventional methods and conditions, or according to the product instructions.

### Example 1: Preparation of 4'-(2-hydroxyethoxy)-4'-(pyrrolidin-1-yl)-3'-(trimethylsilyl)-[1,1':3',1'-terphenyl]-4-carboxylic acid (compound 1-1):

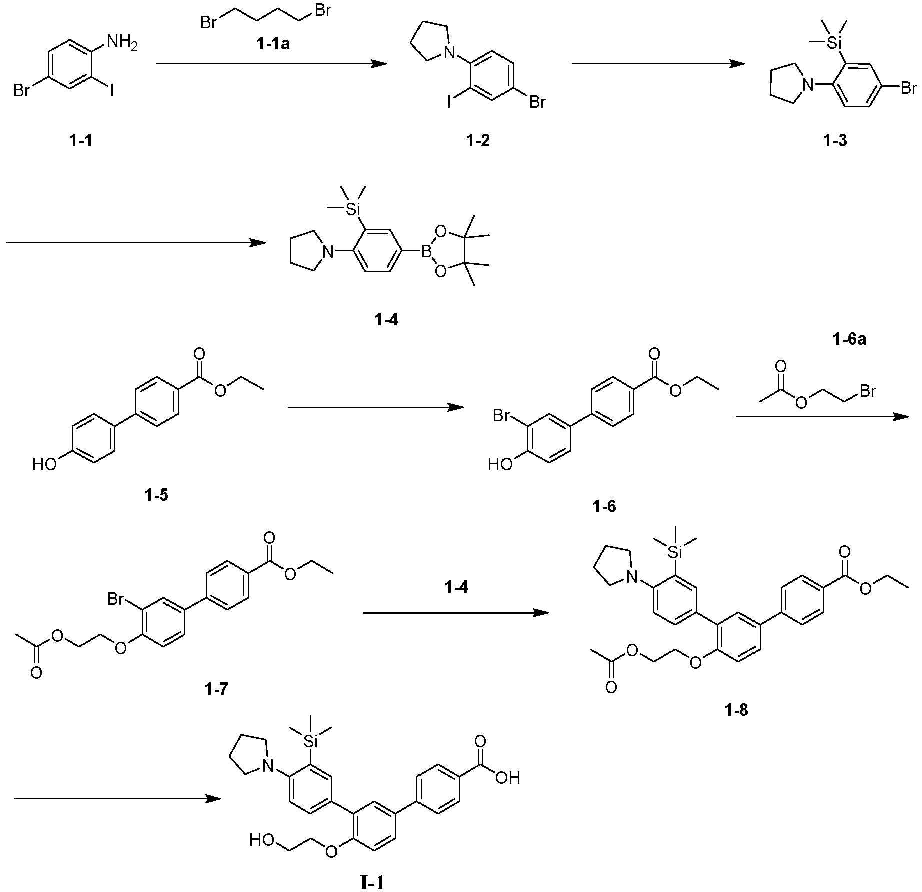

### Step 1: Synthesis of 1-(4-bromo-2-iodophenyl)pyrrolidine (compound 1-2)

4-Bromo-2-iodoaniline (compound **1-1,** 5 g, 16.8 mmol) was dissolved in acetonitrile (50 mL) in a 100 mL single-necked flask, and dibromobutane (7.2 g, 33.6 mmol) and potassium carbonate (7 g, 50.4 mmol) were sequentially added thereto at room temperature. After the addition was complete, the mixture was heated to 80°C in an oil bath and stirred overnight. The solid insoluble material was filtered, then the filtrate was evaporated to dryness by rotary evaporation, and the residue was purified by silica gel column chromatography (mobile phase: petroleum ether) to obtain compound **1-2** (2.6 g, 7.3 mmol) as a yellow oil with a yield of 43.5%.

LCMS [M+1]⁺ = 351.9, 353.9.

### Step 2: Synthesis of 1-(4-bromo-2-(trimethylsilyl)phenyl)pyrrolidine (compound 1-3)

1-(4-Bromo-2-iodophenyl)pyrrolidine (compound **1-2,** 2.6 g, 7.3 mmol) was added into a 100 mL three-necked flask and dissolved in tetrahydrofuran (30 mL), and the mixture was stirred for 5 minutes in a dry ice acetone bath (-78°C). A mixture of n-butyllithium (0.5 mL, 2.5 M) in tetrahydrofuran was added slowly dropwise to the solution under nitrogen atmosphere, and after the dropwise addition was complete, the reaction mixture was continuously stirred and reacted for 1 hour at -78°C. Then, chlorotrimethylsilane (1.6 g, 14.7 mmol) was added slowly dropwise to the reaction mixture, and the temperature was kept constant at -78°C. After the dropwise addition was complete, the reaction mixture was slowly warmed to room temperature while stirring and stirred overnight. The reaction was quenched by saturated ammonium chloride aqueous solution (10 mL), and the reaction mixture was extracted with ether (3 × 20 mL). The organic phases were combined, washed with saturated brine (1 × 10 mL), dried over anhydrous sodium sulfate, filtered, and the filtrate was evaporated to dryness by rotary evaporation to obtain a light yellow residue. The residue was purified by silica gel column chromatography (ethyl acetate: petroleum ether = 1:20) to obtain compound **1-3** (1.2 g, 4.0 mmol) as a light yellow oil with a yield of 54.5%.

LCMS [M+1]⁺ = 298.0, 300.0.

¹H NMR (CDCl₃, 300 MHz) δ 7.55 (d, *J* = 3 Hz, 1H), 7.45 (dd, *J* = 9 Hz, 3Hz, 1H), 7.55 (d, *J=* 9 Hz, 1H), 3.02 - 2.96 (m, 4H), 1.98 - 1.88 (m, 4H), 0.30 (s, 1H).

### Step 3: Synthesis of 1-(4-(4,4,5,5-tetramethyl-1,3,2-dioxaborolan-2-yl)-2-(trimethylsilyl)phenyl)pyrrole (compound 1-4)

1-(4-Bromo-2-(trimethylsilyl)phenyl)pyrrolidine (compound **1-3,** 210 mg, 0.7 mmol), bis(pinacolato)diboron (200 mg, 0.8 mmol), potassium acetate (186 mg, 2.0 mmol), and Pd(dppf)Cl₂ (16 mg, 0.02 mmol) were added into a 10 mL microwave tube. And dioxane (4 mL) was added into the tube. The microwave tube was sealed under nitrogen atmosphere, then heated, and reacted at 120°C for 1 hour. After the reaction was complete, the reaction mixture was diluted with petroleum ether (9 mL), separated by flash column chromatography with a small amount of silica gel layer, and filtered to remove inorganic salts and palladium black. The filtrate was concentrated to obtain compound **1-4** (242 mg) as a light brown crude product. The crude product was used directly in the next reaction step without purification.

LCMS [M+1]⁺ = 346.2.

¹H NMR (DMSO-*d₆*, 800 MHz) δ 7.72 (d, *J=* 1.5 Hz, 1 H), 7.58 (dd, *J* = 1.6, 8.1 Hz, 1 H), 7.04 (d, *J =* 8.1 Hz, 1 H), 3.03 (t, *J* = 6.4 Hz, 4 H), 1.86 (td, *J* = 3.2, 6.4 Hz, 4 H), 1.26 (s, 12 H), 0.24 (s, 9 H).

### Step 4: Synthesis of ethyl 3'-bromo-4'-hydroxy-[1,1'-biphenyl]-4-carboxylate (compound 1-6)

Ethyl 4'-hydroxy-[1,1'-biphenyl]-4-carboxylate (compound **1-5,** 1 g, 4.1 mmol) was dissolved in DCM (15 mL) in a 50 mL single-necked flask. NBS (728 mg, 4.1 mmol) was added thereto in five batches at 25°C, and after the addition was complete, the reaction mixture was stirred for another 1 hour at room temperature. After the stirring was complete, the reaction mixture was concentrated, and the crude product was purified by column chromatography (petroleum ether: ethyl acetate = 10:1 to 1:1) to obtain compound **1-6** (1.1 g) as a white solid with a yield of 83.5%.

LCMS [M+1]⁺ = 321.0, 323.0.

### Step 5: Synthesis of ethyl 4'-(2-acetoxyethoxy)-3'-bromo-[1,1'-biphenyl]-4-carboxylate (compound 1-7)

Ethyl 3'-bromo-4'-hydroxy-[1,1'-biphenyl]-4-carboxylate (compound **1-6,** 1.1 g, 3.4 mmol) was placed in a 50 mL single-necked flask and dissolved in DMF (10 mL). NaH (163 mg, 60%, 1.2 eq) was added thereto slowly in an ice water bath at 0°C. The reaction mixture was stirred and reacted for 0.5 hours, and then added dropwise with 2-bromoethyl acetate (678 mg, 4.1 mmol). After the dropwise addition was complete, the reaction mixture was stirred overnight. The system was then added with 30 mL of water, extracted with ethyl acetate, dried over anhydrous sodium sulfate, filtered, concentrated, and purified by silica gel column chromatography (petroleum ether: ethyl acetate = 100:1 to 1:1) to obtain compound **1-7** (1.0 g) as a white solid with a yield of 72.2%.

¹H NMR (DMSO-d₆, 800 MHz) δ 8.02 - 8.00 (m, 2 H), 7.98 (d, *J* = 2.3 Hz, 1 H), 7.84 - 7.81 (m, *J* = 8.4 Hz, 2 H), 7.75 (dd, *J* = 2.3, 8.6 Hz, 1 H), 7.27 (d, *J* = 8.7 Hz, 1 H), 4.42 - 4.38 (m, 2 H), 4.36 - 4.31 (m, 4 H), 2.06 (s, 3 H), 1.34 (t, *J=* 7.2 Hz, 3 H).

### Step 6: Synthesis of ethyl 4'-(2-acetoxyethoxy)-4'-(pyrrolidin-1-yl)-3'-(trimethylsilyl)-[1,1':3',1'-terphenyl]-4-carboxylate (compound 1-8)

Compound **1-4** (242 mg, 0.7 mmol), compound **1-7** (284 mg, 0.7 mmol), sodium bicarbonate (168 mg, 2.0 mmol), and Pd(dppf)Cl₂ (16 mg, 0.02 mmol) were added into a 10 mL microwave tube. The system was added with dioxane (4 mL) and H₂O (1 mL). The microwave tube was sealed under nitrogen atmosphere, and the reaction mixture was reacted at 120°C for 1 hour under microwave irradiation. After the reaction was complete, the reaction mixture was diluted with petroleum ether (9 mL) and ethyl acetate (2 mL). The reaction mixture was then dried over anhydrous sodium sulfate, and filtered by a small amount of silica gel layer to remove inorganic salts and palladium black. The filtrate was concentrated to obtain compound **1-8** (382 mg) as a light brown crude product, which was used directly in the next step without further purification.

LCMS [M+1]⁺ = 546.2.

### Step 7: Synthesis of 4'-(2-hydroxyethoxy)-4'-(pyrrolidin-1-yl)-3'-(trimethylsilyl)-[1,1':3',1'-terphenyl]-4-carboxylic acid (compound 1-1)

Ethyl 4'-(2-acetoxyethoxy)-4'-(pyrrolidin-1-yl)-3'-(trimethylsilyl)-[1,1':3',1'-terphenyl]-4-carboxylate (compound **1-8,** 382 mg, 0.7 mmol) was placed in a 25 mL single-necked flask. Compound **1-8** was dissolved in tetrahydrofuran (3 mL). NaOH aqueous solution (2 M, 1 mL) was added to the reaction system. The reaction system was stirred for 2 hours at 40°C. After the reaction was complete, the reaction system was slowly adjusted to neutrality with formic acid. The reaction mixture was directly purified by reversed-phase column chromatography (H₂O/MeCN, 30% to 100%, 0.05% FA), and the resulting eluate was concentrated until a large amount of solid precipitated, filtered, washed with acetonitrile (1 mL × 2), and dried to obtain compound **1-1** (87 mg) as a white solid product with a yield of 26.2%.

LCMS [M+1]⁺ = 476.2.

¹H NMR (DMSO-*d₆,* 800 MHz) δ 7.98 - 7.96 (m, 2 H), 7.81 - 7.79 (m, 2 H), 7.66 - 7.64 (m, 2 H), 7.61 - 7.58 (m, 2 H), 7.28 (d, *J=* 8.3 Hz, 1 H), 7.22 (d, *J=* 8.7 Hz, 1 H), 4.09 (t, *J=* 5.2 Hz, 2 H), 3.72 - 3.69 (m, 2 H), 2.99 (t, *J=* 5.4 Hz, 4 H), 1.88 (td, *J* = 3.1, 6.3 Hz, 4 H), 0.28 - 0.26 (m, 9 H).

### Example 2: Preparation of 4'-(3-hydroxypropoxy)-4"-(pyrrolidin-1-yl)-3"-(trimethylsilyl)-[1,1':3',1"-terphenyl]-4-carboxylic acid (compound 1-2):

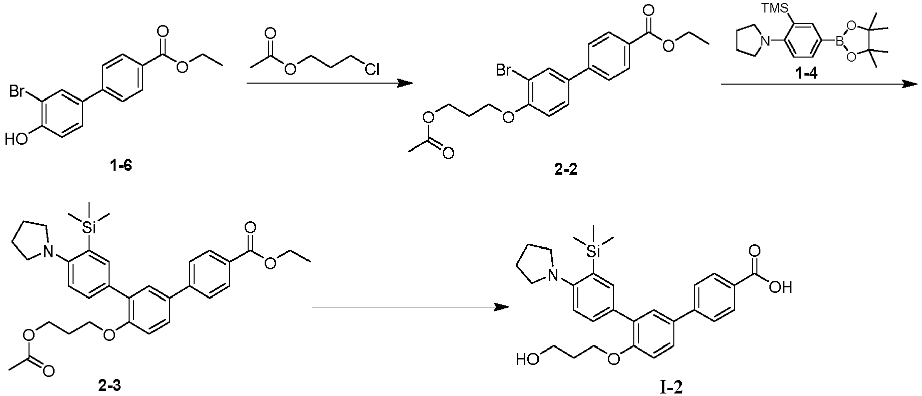

### Step 1: Synthesis of ethyl 4'-(3-acetoxypropoxy)-3'-bromo-[1,1'-biphenyl]-4-carboxylate (compound 2-2)

The reaction flask was added with ethyl 3'-bromo-4'-hydroxy-[1,1'-biphenyl]-4-carboxylate (compound **1-6,** 110 mg, 0.34 mmol), acetonitrile (4 mL), 3-chloropropyl acetate (136 mg, 1 mmol), potassium carbonate (138 mg, 1 mmol), and a catalytic amount of potassium iodide, and then the reaction mixture was stirred for 12 hours at 80°C. The reaction mixture was filtered, concentrated, and purified by silica gel column chromatography (petroleum ether: ethyl acetate = 100:1 to 1:1) to obtain compound **2-2** (112 mg) as a white solid with a yield of 78.2%.

LCMS [M+1]⁺ = 421.0, 423.0.

### Step 2: Synthesis of ethyl 4'-(3-acetoxypropoxy)-4"-(pyrrolidin-1-yl)-3"-(trimethylmethylsilyl)-[1,1':3',1"-terphenyl]-4-carboxylate (compounds 2-3)

Compound **1-4** (60 mg, 0.17 mmol), compound **2-2** (71.4 mg, 0.17 mmol), sodium bicarbonate (42 mg, 0.5 mmol), and Pd(dppf)Cl₂ (8 mg, 0.01 mmol) were added into a 10 mL microwave tube. The system was added with dioxane (4 mL) and H₂O (1 mL). The microwave tube was sealed under nitrogen atmosphere, and the reaction mixture was reacted at 120°C for 1 hour under microwave irradiation. After the reaction was complete, the reaction mixture was diluted with petroleum ether (9 mL) and ethyl acetate (2 mL). The reaction mixture was then dried over anhydrous sodium sulfate, and filtered by a small amount of silica gel layer to remove inorganic salts and palladium black. The filtrate was concentrated to obtain compound **2-3** (90 mg) as light brown crude product, which was used directly in the next step without further purification.

LCMS [M+1]⁺ = 560.2.

### Step 3: Synthesis of 4'-(3-hydroxypropoxy)-4"-(pyrrolidin-1-yl)-3"-(trimethylmethylsilyl)-[1,1':3',1"-terphenyl]-4-carboxylic acid (compound 1-2)

Ethyl 4'-(3-acetoxypropoxy)-4"-(pyrrolidin-1-yl)-3"-(trimethylmethylsilyl)-[1,1':3',1"-terphenyl]-4-carboxylate (compound **2-3,** 90 mg of the crude product) was placed in a 25 mL single-necked flask. Compound **2-3** was dissolved in tetrahydrofuran (3 mL). NaOH aqueous solution (1 M, 1 mL) was added to the reaction system. The reaction system was stirred for 2 hours at 40°C. After the reaction was complete, the reaction system was slowly adjusted to neutrality with formic acid. The reaction mixture was directly purified by reversed-phase column chromatography (H₂O/MeCN, 30% to 100%, 0.05% FA), and the resulting eluate was concentrated until a large amount of solid precipitated, filtered, washed with acetonitrile (1 mL × 2), and dried to obtain compound **1-2** (19 mg) as a white solid product.

LCMS [M+1]⁺ = 490.2.

¹H NMR (800 MHz, DMSO-d₆) δ 8.00 - 7.96 (m, *J* = 8.5 Hz, 2 H), 7.83 - 7.80 (m, *J* = 8.5 Hz, 2 H), 7.68 (dd, *J* = 2.4, 8.5 Hz, 1 H), 7.62 (dd, *J* = 2.3, 9.7 Hz, 2 H), 7.55 (dd, *J* = 2.2, 8.3 Hz, 1 H), 7.30 (d, *J =* 8.3 Hz, 1 H), 7.21 (d, *J =* 8.7 Hz, 1 H), 4.12 (t, *J =* 6.3 Hz, 2 H), 3.53 (t, *J=* 6.2 Hz, 2 H), 3.01 (br. s., 4 H), 1.89 (br. s., 4 H), 1.85 (t, *J=* 6.3 Hz, 2 H), 0.28 (s, 9 H).

### Example 3: Preparation of 4'-(4-hydroxybutoxy)-4"-(pyrrolidin-1-yl)-3"-(trimethylsilyl)-[1,1':3',1"-terphenyl]-4-carboxylic acid (compound 1-3):

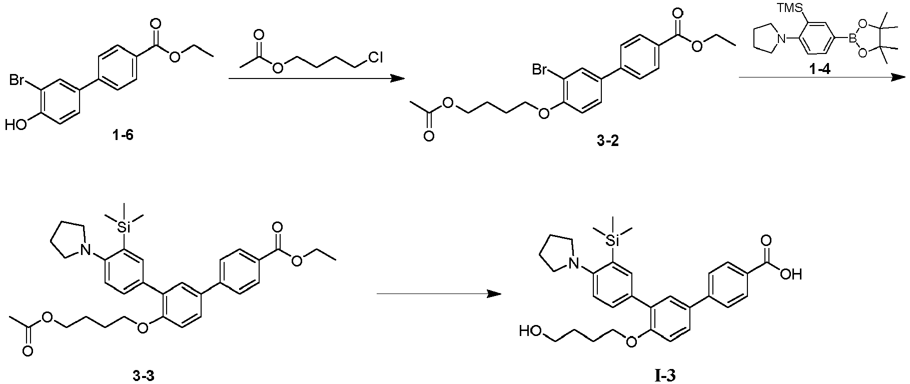

### Step 1: Synthesis of ethyl 4'-(4-acetoxybutoxy)-3'-bromo-[1,1'-biphenyl]-4-carboxylate (compound 3-2)

The reaction flask was added with ethyl 3'-bromo-4'-hydroxy-[1,1'-biphenyl]-4-carboxylate (compound **1-6,** 110 mg, 0.34 mmol), acetonitrile (4 mL), 4-chlorobutyl acetate (150 mg, 1 mmol), potassium carbonate (138 mg, 1 mmol), and a catalytic amount of potassium iodide, and then the reaction mixture was stirred for 12 hours at 80°C. The reaction mixture was filtered, concentrated, and purified by silica gel column chromatography (petroleum ether: ethyl acetate = 100:1 to 1:1) to obtain compound **3-2** (103 mg) as a white solid with a yield of 70%.

LCMS [M+1]⁺ = 435.0, 437.0.

### Step 2: Synthesis of ethyl 4'-(4-acetoxybutoxy)-4"-(pyrrolidin-1-yl)-3"-(trimethylsilyl)-[1,1':3',1"-terphenyl]-4-carboxylate (compound 3-3)

Compound **1-4** (60 mg, 0.17 mmol), compound **3-2** (73.9 mg, 0.17 mmol), sodium bicarbonate (42 mg, 0.5 mmol), and Pd(dppf)Cl₂ (8 mg, 0.01 mmol) were added into a 10 mL microwave tube. The system was added with dioxane (4 mL) and H₂O (1 mL). The microwave tube was sealed under nitrogen atmosphere, and the reaction mixture was reacted at 120°C for 1 hour under microwave irradiation. After the reaction was complete, the reaction mixture was diluted with petroleum ether (9 mL) and ethyl acetate (2 mL). The reaction mixture was then dried over anhydrous sodium sulfate, and filtered by a small amount of silica gel layer to remove inorganic salts and palladium black. The filtrate was concentrated to obtain compound **3-3** (88 mg) as a light brown crude product, which was used directly in the next step without further purification.

LCMS [M+1]⁺ = 574.2.

### Step 3: Synthesis of 4'-(4-hydroxybutoxy)-4"-(pyrrolidin-1-yl)-3"-(trimethylsilyl)-[1,1':3',1"-terphenyl]-4-carboxylic acid (compound 1-3)

Ethyl 4'-(4-acetoxybutoxy)-4"-(pyrrolidin-1-yl)-3"-(trimethylsilyl)-[1,1':3',1"-terphenyl]-4-carboxylate (compound **3-3,** 88 mg of the crude product) was placed in a 25 mL single-necked flask. Compound **3-3** was dissolved in tetrahydrofuran (3 mL). NaOH aqueous solution (1 M, 1 mL) was added to the reaction system. The reaction system was stirred for 2 hours at 40°C. After the reaction was complete, the reaction system was slowly adjusted to neutrality with formic acid. The reaction mixture was directly purified by reversed-phase column chromatography (H₂O/MeCN, 30% to 100%, 0.05% FA), and the resulting eluate was concentrated until a large amount of solid precipitated, filtered, washed with acetonitrile (1 mL × 2), and dried to obtain compound **1-3** (26 mg) as a white solid product.

LCMS [M+1]⁺ = 504.2.

¹H NMR (800 MHz, DMSO-d₆) = 8.04 - 7.95 (m, *J* = 8.4 Hz, 2 H), 7.83 - 7.78 (m, *J* = 8.5 Hz, 2 H), 7.67 (dd, *J* = 2.4, 8.5 Hz, 1 H), 7.61 (d, *J* = 2.4 Hz, 1 H), 7.63 (d, *J* = 2.2 Hz, 1 H), 7.54 (dd, *J* = 2.2, 8.3 Hz, 1 H), 7.31 (d, *J =* 8.4 Hz, 1 H), 7.21 (d, *J =* 8.7 Hz, 1 H), 4.06 (t, *J* = 6.5 Hz, 2 H), 3.42 (t, *J* = 6.4 Hz, 2 H), 3.00 (br. s., 4 H), 1.89 (t, *J* = 2.9 Hz, 4 H), 1.76 - 1.72 (m, 2 H), 1.56 - 1.51 (m, 2 H), 0.28 - 0.26 (s, 9 H).

### Example 4: Preparation of 4'-(diethylamino)-4'-(2-hydroxyethoxy)-3'-(trimethylsilyl)-[1,1':3',1"-terphenyl]-4-carboxylic acid (compound 1-4):

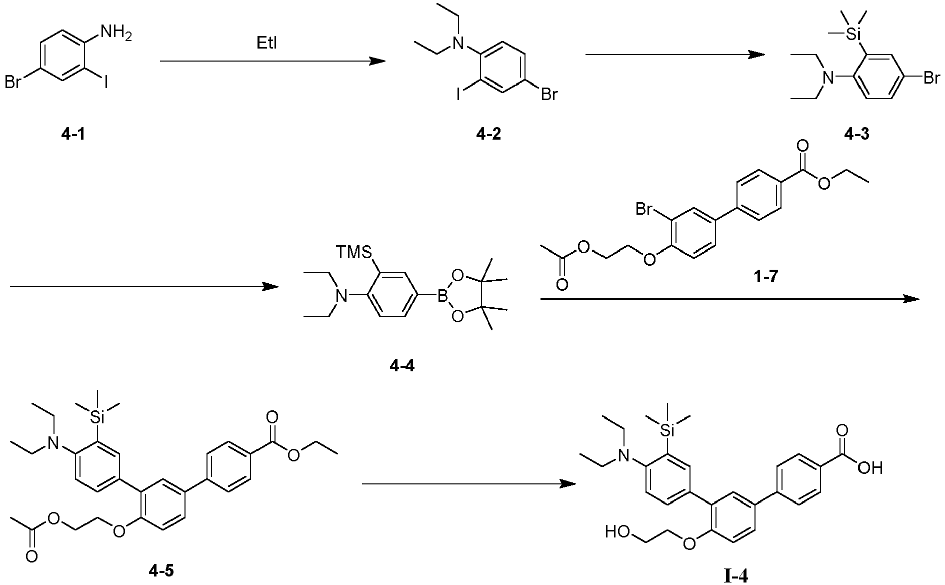

### Step 1: Synthesis of 4-bromo-N,N-diethyl-2-iodoaniline (compound 4-2)

4-Bromo-2-iodoaniline (compound **4-1,** 0.9 g, 3 mmol) was dissolved in acetonitrile (20 mL) in a 50 mL sealed tube, and iodoethane (3.1 g, 20 mmol) and potassium carbonate (810 mg, 6 mmol) were sequentially added thereto at room temperature, and after the addition was complete, the flask was sealed tightly, and the mixture was stirred for 12 hours at 80°C. The solid insoluble material was filtered, then the filtrate was evaporated to dryness by rotary evaporation, and the residue was purified by silica gel column chromatography (mobile phase: petroleum ether: ethyl acetate = 100 to 20:1) to obtain compound **4-2** (350 mg, 1.0 mmol) as a yellow oil with a yield of 33%.

LCMS [M+1]⁺ = 353.9, 355.9.

### Step 2: Synthesis of 4-bromo-N,N-diethyl-2-(trimethylsilyl)aniline (compound 4-3)

4-Bromo-*N,N-*diethyl-2-iodoaniline (compound **4-2,** 350 mg, 1.0 mmol) was added into a 50 mL three-necked flask, dissolved in tetrahydrofuran (10 mL), and the mixture was stirred for 5 minutes in a dry ice acetone bath (-78°C). *n*-Butyllithium (0.5 mL, 2.5 M) was added slowly dropwise to the solution under nitrogen atmosphere, and after the dropwise addition was complete, the reaction mixture was continuously stirred and reacted for 1 hour at -78°C. Then, chlorotrimethylsilane (180 mg, 1.7 mmol) was added slowly dropwise to the reaction mixture, and the temperature was kept constant at -78°C. After the dropwise addition was complete, the reaction mixture was slowly warmed to room temperature while stirring and stirred overnight. The reaction was quenched by saturated ammonium chloride aqueous solution (10 mL), and the reaction mixture was extracted with ether (3 × 20 mL). The organic phases were combined, washed with saturated brine (1 × 10 mL), dried over anhydrous sodium sulfate, filtered, and the filtrate was evaporated to dryness by rotary evaporation to obtain a light yellow residue. The residue was purified by silica gel column chromatography (ethyl acetate: petroleum ether = 1:20) to obtain compound **4-3** (150 mg, 0.5 mmol) as a light yellow oil with a yield of 50%.

LCMS [M+1]⁺ = 300.0, 302.0.

### Step 3: Synthesis of N,N-diethyl-4-(4,4,5,5-tetramethyl-1,3,2-dioxaborolan-2-yl)-2-(trimethylsilyl)aniline (compound 4-4)

4-Bromo-N,N-diethyl-2-(trimethylsilyl)aniline (compound **4-3,** 150 mg, 0.5 mmol), bis(pinacolato)diboron (153 mg, 0.6 mmol), potassium acetate (98 mg, 1.0 mmol), and Pd(dppf)Cl₂ (8 mg, 0.01 mmol) were added into a 10 mL microwave tube. And dioxane (4 mL) was added into the tube. The microwave tube was sealed under nitrogen atmosphere, then heated, and reacted at 120°C for 1 hour. After the reaction was complete, the reaction mixture was diluted with petroleum ether (9 mL), separated by flash column chromatography with a small amount of silica gel layer, and filtered to remove inorganic salts and palladium black. The filtrate was concentrated to obtain compound **4-4** (180 mg) as a light brown crude product. The crude product was used directly in the next reaction step without purification.

LCMS [M+1]⁺ = 348.2.

### Step 4: Synthesis of ethyl 4'-(2-acetoxyethoxy)-4'-(diethylamino)-3'-(trimethylsilyl)-[1,1':3',1"-terphenyl]-4-carboxylate (compound 4-5)

Compound **4-4** (60 mg, 0.17 mmol), compound **1-7** (68 mg, 0.17 mmol), sodium bicarbonate (42 mg, 0.5 mmol), and Pd(dppf)Cl₂ (8 mg, 0.01 mmol) were added into a 10 mL microwave tube. The system was added with dioxane (4 mL) and H₂O (1 mL). The microwave tube was sealed under nitrogen atmosphere, and the reaction mixture was reacted at 120°C for 1 hour under microwave irradiation. After the reaction was complete, the reaction mixture was diluted with petroleum ether (9 mL) and ethyl acetate (2 mL). The reaction mixture was then dried over anhydrous sodium sulfate, and filtered by a small amount of silica gel layer to remove inorganic salts and palladium black. The filtrate was concentrated to obtain compound **4-5** (80 mg) as a light brown crude product, which was used directly in the next step without further purification.

LCMS [M+1]⁺ = 548.2.

### Step 5: Synthesis of 4'-(diethylamino)-4'-(2-hydroxyethoxy)-3'-(trimethylsilyl)-[1,1':3',1"-terphenyl]-4-carboxylic acid (compound 1-4)

Ethyl 4'-(2-acetoxyethoxy)-4'-(diethylamino)-3'-(trimethylsilyl)-[1,1':3',1"-terphenyl]-4-carboxylate (compound **4-5, 80** mg of the crude product) was placed in a 25 mL single-necked flask. Compound **4-5** was dissolved in tetrahydrofuran (3 mL). NaOH aqueous solution (1 M, 1 mL) was added to the reaction system. The reaction system was stirred for 2 hours at 40°C. After the reaction was complete, the reaction system was slowly adjusted to neutrality with formic acid. The reaction mixture was directly purified by reversed-phase column chromatography (H₂O/MeCN, 30% to 100%, 0.05% FA), and the resulting eluate was concentrated until a large amount of solid precipitated, filtered, washed with acetonitrile (1 mL × 2), and dried to obtain compound **1-4** (17 mg) as a white solid product.

LCMS [M+1]⁺ = 478.2.

¹H NMR (DMSO-d₆, 800 MHz) δ 7.93 - 7.90 (m, 2H), 7.80 - 7.77 (m, 2H), 7.65 - 7.63 (m, 2H), 7.60 - 7.58 (m, 2H), 7.26 (d, *J* = 8.2 Hz, 1H), 7.20 (d, *J* = 8.5 Hz, 1H), 4.08 (t, *J=* 5.2 Hz, 2H), 3.70 - 3.67 (m, 2H), 3.05 - 2.94 (m, 4H), 1.12 - 1.04 (m, 6H), 0.27 - 0.25 (m, 9H).

### Example 5: Preparation of 4'-(2-hydroxyethoxy)-3'-(1,1,4,4-tetramethyl-1,2,3,4-tetrahydrobenzo[b][1,4]disilan-6-yl)-[1,1'-biphenyl]-4-carboxylic acid (compound 1-5):

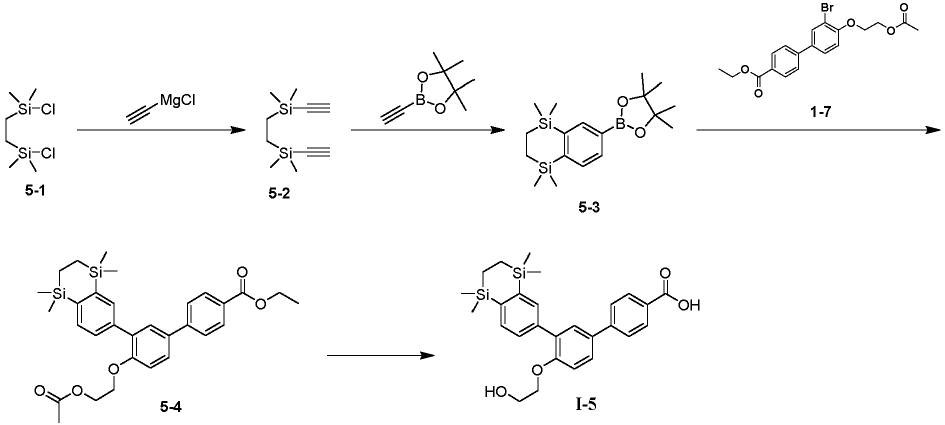

### Step 1: Synthesis of 1,2-bis(ethyldimethylsilyl)ethane (compound 5-2)

1,2-Bis(chlorodimethylsilyl)ethane (compound **5-1,** 3.00 g, 13.94 mmol) was taken and dissolved in a 30 mL solution of anhydrous tetrahydrofuran, and the system was charged with argon. Ethynylmagnesium chloride (70 mL, 0.5 mol/L, 35.00 mmol) was added slowly dropwise thereto at room temperature. The reaction mixture was stirred at room temperature for 1 hour and then refluxed for 1 hour. After the reaction was complete, the reaction mixture was added with saturated ammonium chloride solution to quench. The reaction system was added with an appropriate amount of ethyl acetate and washed twice with saturated brine, and the organic phases were collected and dried over anhydrous sodium sulfate. The organic phase was filtered, evaporated to dryness under reduced pressure, and the crude product was purified by silica gel column chromatography (petroleum ether) to obtain compound **5-2** (1.80 g, 9.26 mmol) as a colorless liquid with a yield of 66%.

¹H NMR (300 MHz, CDCl₃) δ 2.37 (s, 2H), 0.61 (s, 4H), 0.18 (s, 12H).

### Step 2: Synthesis of 1,1,4,4-tetramethyl-6-(4,4,5,5-tetramethyl-1,3,2-dioxaborolan-2-yl)-1,2,3,4-tetrahydrobenzo[b][1,4]diphenylamine (compound 5-3)

Iodine (20.00 mg, 0.08 mmol) was dissolved in 10 mL of anhydrous acetonitrile, then zinc (45.00 mg, 0.69 mmol) was added thereto, and the mixture was stirred at room temperature until the yellow color disappeared and a gray suspension appeared. The system was charged with argon, and compound **5-2** (1.38 g, 7.10 mmol), 2-ethynyl-4,4,5,5-tetramethyl-[1,3,2]dioxaborolane (1.40 mg, 9.21 mmol), and a 0.1 M solution of cobalt(II) iodide (2.00 mL, 0.20 mmol) were sequentially added thereto at room temperature. The system was switched to react at 50°C, monitored by TLC, and the reaction was terminated when compound **5-2** was completely consumed. The system was initially purified by silica gel column chromatography (ether: *n*-hexane = 4:1), then the organic phases were combined, and the solvent was evaporated to dryness under reduced pressure to obtain a crude product as a yellow liquid. The crude product was purified again by silica gel column chromatography (n-hexane) to obtain compound **5-3** (0.50 g) as a colorless crystal with a yield of 20.3%.

¹H NMR (300 MHz, CDCl₃) δ 7.84 (s, 1 H), 7.68 (br s, 1 H), 7.41 (br s, 1 H), 1.22 (s, 12 H), 0.90 (s, 4 H), 0.16 (s, 6 H), 0.12 (s, 6 H).

### Step 3: Synthesis of ethyl 4'-(2-acetoxyethoxy)-3'-(1,1,4,4-tetramethyl-1,2,3,4-tetrahydrobenzo[b][1,4]disilan-6-yl)-[1,1'-biphenyl]-4-carboxylate (compound 5-4)

Compound **1-7** (61 mg, 0.15 mmol), compound **5-3** (52 mg, 0.15 mmol), sodium bicarbonate (42 mg, 0.5 mmol), and Pd(dppf)Cl₂ (8 mg, 0.01 mmol) were added into a 10 mL microwave tube. The system was added with dioxane (4 mL) and H₂O (1 mL). The microwave tube was sealed under nitrogen atmosphere, and the reaction mixture was reacted at 120°C for 1 hour under microwave irradiation. After the reaction was complete, the reaction mixture was diluted with petroleum ether (2 mL) and ethyl acetate (9 mL). The reaction mixture was dried over anhydrous sodium sulfate, concentrated, and purified by silica gel column chromatography (petroleum ether/ethyl acetate = 100 to 10/1) to obtain compound **5-4** (70 mg) as a colorless oil with a yield of 85.3%.

LCMS [M+1]⁺ = 547.2.

¹H NMR (800 MHz, DMSO-d₆) δ 7.78 (d, *J* = 8.4, 2 H), 7.63 (d, *J* = 8.4, 2 H),, 7.52 (d, *J =* 1.2 Hz, 1 H), 7.50 (dd, *J* = 2.5, 8.5 Hz, 1 H), 7.43 (d, *J =* 2.4 Hz, 1 H), 7.37 - 7.31 (m, 2 H), 7.05 (d, *J =* 8.6 Hz, 1 H), 4.11 (q, *J =* 7.1 Hz, 2 H), 4.09 - 4.05 (m, 4 H), 1.76 (s, 3 H), 1.11 (t, *J* = 7.1 Hz, 3 H), 0.78 (s, 4 H), 0.02-0.02 (m, 12 H).

### Step 4: Synthesis of 4'-(2-hydroxyethoxy)-3'-(1,1,4,4-tetramethyl-1,2,3,4-tetrahydrobenzo[b][1,4]disilan-6-yl)-[1,1'-biphenyl]-4-carboxylic acid (compound 1-5)

Ethyl 4'-(2-acetoxyethoxy)-3'-(1,1,4,4-tetramethyl-1,2,3,4-tetrahydrobenzo[b][1,4]disilan-6-yl)-[1,1'-biphenyl]-4-carboxylate (compound 5-4, 70 mg, 0.128 mmol) was placed in a 25 mL single-necked flask. Compound 5-4 was dissolved in tetrahydrofuran (3 mL). NaOH aqueous solution (1 M, 1 mL) was added to the reaction system. The reaction system was stirred for 2 hours at 40°C. After the reaction was complete, the reaction system was slowly adjusted to neutrality with formic acid. The reaction mixture was extracted with ethyl acetate (20 mL), dried over anhydrous sodium sulfate, concentrated to about 1 mL of solvent, and then added with 10 mL of acetonitrile. When a large amount of solid precipitated, the mixture was filtered and dried to obtain **1-5** (21 mg) as a white solid with a yield of 34.4%.

LCMS [M+1]⁺ = 477.2.

¹H NMR (800 MHz, DMSO-*d₆*) δ 7.74 (d, *J* = 8.4 Hz, 2 H), 7.59 - 7.53 (m, 3 H), 7.45 (dd, *J* = 2.4, 8.6 Hz, 1 H), 7.42 - 7.36 (m, 1 H), 7.36 - 7.30 (m, 2 H), 7.00 (d, *J=* 8.6 Hz, 1 H), 3.87 (t, *J* = 5.1 Hz, 2 H), 3.48 (t, *J* = 5.1 Hz, 2 H), 0.76 (s, 4 H), 0.03 - 0.02 (m, 12 H).

### Example 6: Preparation of 4'-(3-hydroxypropoxy)-3'-(1,1,4,4-tetramethyl-1,2,3,4-tetrahydrobenzo[b][1,4]disilan-6-yl)-[1,1'-biphenyl]-4-carboxylic acid (compound 1-6):

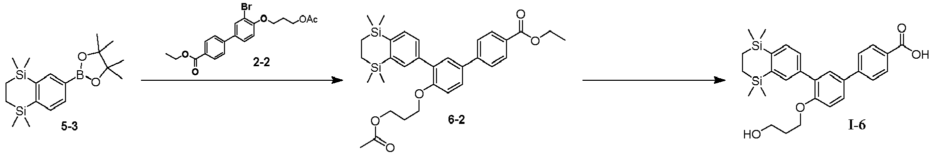

### Step 1: Synthesis of ethyl 4'-(3-acetoxypropoxy)-3'-(1,1,4,4-tetramethyl-1,2,3,4-tetrahydrobenzo[b][1,4]disilan-6-yl)-[1,1'-biphenyl]-4-carboxylate (compound 6-2)

Compound **2-2** (63 mg, 0.15 mmol), compound **5-3** (52 mg, 0.15 mmol), sodium bicarbonate (42 mg, 0.5 mmol), and Pd(dppf)Cl₂ (8 mg, 0.01 mmol) were added into a 10 mL microwave tube. The system was added with dioxane (4 mL) and H₂O (1 mL). The microwave tube was sealed under nitrogen atmosphere, and the reaction mixture was reacted at 120°C for 1 hour under microwave irradiation. After the reaction was complete, the reaction mixture was diluted with petroleum ether (2 mL) and ethyl acetate (9 mL). The reaction mixture was dried over anhydrous sodium sulfate, concentrated, and purified by silica gel column chromatography (petroleum ether/ethyl acetate = 100 to 10/1) to obtain compound **6-2** (75 mg) as a colorless oil with a yield of 89.2%.

LCMS [M+1]⁺ = 561.2.

¹H NMR (800 MHz, DMSO-d₆) δ 7.79 - 7.78 (m, 2 H), 7.64 - 7.62 (m, 2 H), 7.51 - 7.49 (m, 2 H), 7.41 (d, *J =* 2.5 Hz, 1 H), 7.35 (d, *J* = 7.6 Hz, 1 H), 7.30 (dd, *J =* 1.8, 7.6 Hz, 1 H), 7.03 (d, *J =* 8.7 Hz, 1 H), 4.11 (q, *J =* 7.2 Hz, 2 H), 3.91 (t, *J =* 6.2 Hz, 2 H), 3.88 (t, *J =* 6.4 Hz, 2 H), 1.80 - 1.78 (m, 2 H), 1.73 (s, 3 H), 1.12 (t, *J =* 7.1 Hz, 3 H), 0.78 (s, 4 H), 0.02 (s, 6 H), 0.00 (s, 6 H).

### Step 2: Synthesis of 4'-(3-hydroxypropoxy)-3'-(1,1,4,4-tetramethyl-1,2,3,4-tetrahydrobenzo[b][1,4]disilan-6-yl)-[1,1'-biphenyl]-4-carboxylic acid (compound 1-6)

Ethyl 4'-(3-acetoxypropoxy)-3'-(1,1,4,4-tetramethyl-1,2,3,4-tetrahydrobenzo[b][1,4]disilan-6-yl)-[1,1'-biphenyl]-4-carboxylate (compound **6-2,** 75 mg, 0.133 mmol) was placed in a 25 mL single-necked flask. Compound **6-2** was dissolved in tetrahydrofuran (3 mL). NaOH aqueous solution (1 M, 1 mL) was added to the reaction system. The reaction system was stirred for 2 hours at 40°C. After the reaction was complete, the reaction system was slowly adjusted to neutrality with formic acid. The reaction mixture was extracted with ethyl acetate (20 mL), dried over anhydrous sodium sulfate, concentrated to about 1 mL of solvent, and then added with 10 mL of acetonitrile. When a large amount of solid precipitated, the mixture was filtered and dried to obtain **1-6** (40 mg) as a white solid with a yield of 61.2%.

LCMS [M+1]⁺ = 491.2.

¹H NMR (800 MHz, DMSO-d₆) δ 7.78 - 7.74 (m, 2 H), 7.61 - 7.58 (m, 2 H), 7.51 (d, *J=* 1.3 Hz, 1 H), 7.50 - 7.47 (m, 1 H), 7.39 (d, *J=* 2.5 Hz, 1 H), 7.37 - 7.33 (m, 1 H), 7.31 (d, *J =* 1.8 Hz, 1 H), 7.01 (d, *J =* 8.7 Hz, 1 H), 3.90 (t, *J =* 6.3 Hz, 2 H), 3.30 (t, *J =* 6.2 Hz, 2 H), 1.62 (t, *J=* 6.3 Hz, 2 H), 0.77 (s, 4 H), 0.02 - 0.02 (m, 12 H).

### Bioassay example 1: Agonist activity of RARγ and RARα

The HEK-293 cell line (ATCC#CRL-1573) was used to test the agonist activity of the compound on RARγ and RARα.

The experimental materials are shown in Table 1:

**Table 1**

| Name of experimental materials | Supplier | Cat. No. |
|---|---|---|
| MEM | Gibco | 11090081 |
| Fetal bovine serum | Biological Industries | 04-001-1A |
| Trypsin-EDTA | Gibco | 25200072 |
| FuGENE HD transfection reagent | Promega | E231A |
| Dual Luciferase Reporter Gene Assay System | Promega | E2940 |
| 96-well plate | Costar | 3903 |

### Experimental steps:

1. Day 1: Cell culture. The culture dish was treated with trypsin, and then cells at an appropriate concentration were inoculated into 10 mL of culture medium. Cell culture conditions: 37°C, 5% CO₂, humid environment.
2. Day 3: Transfection with FuGENE transfection reagent. A transfection mixture was prepared using the method of the supplier's instructions, then the tube was shaken vigorously to mix well, and the transfection mixture was left to incubate for 15 minutes at room temperature. The mixture was treated with trypsin to determine the cell density, and the cell suspension was appropriately diluted to the appropriate volume and density. The appropriate transfection reagent mixture was added thereto, and the cell suspension was added to a 96-well plate at 100 µL per well. The 96-well plate was incubated for 24 hours under the cell culture conditions in step 1.
3. Day 4: Addition of test compound. The compound was formulated into a DMSO stock solution at a concentration of 210x and diluted to make a solution at a concentration of 21x by the addition of 9 times the amount of solvent. 5 µL of the compound solution at a concentration of 21x was added to each well and incubated for 24 hours under the same conditions as step 1.
4. Day 5: Plate reading. The Dual Luciferase Reporter Gene Assay System was left at room temperature for 30 minutes prior to use, and Fluorescent Reagent II was added to each well of the 96-well plate to detect the intensity of firefly luciferin in an appropriate manner. Afterwards, Stop&Glo reagent was added to detect the intensity of Renilla luciferin.
5. Data processing. Signal = intensity of firefly luciferin/intensity of Renilla luciferin, multiple of agonist activation = compound signal/baseline signal (the intensity of the baseline signal was obtained from DMSO solution without the compound). pEC₅₀ values were obtained by processing the data with GraphPad Prism, which were further converted to EC₅₀ values.

The experimental results of some compounds of the present disclosure are shown in Table 2.

**Table 2**

| **Compound** | **RARγ, EC₅₀** | **RARα, EC₅₀** |
|---|---|---|
| **I-1** | 0.88 nM | 63.97 nM |
| **I-2** | 0.77 nM | 15.49 nM |
| **I-3** | 9.08 nM | 381.9 nM |
| **I-5** | 1.05 nM | 18.49 nM |
| **Trifarotene** | 1.03 nM | 14.86 nM |

It can be seen from the data in Table 2 that the agonist activity of compounds I-1, I-2, and I-5 on RARγ is equal to or stronger than that of Trifarotene, and the selectivity of compounds 1-1, I-2, I-3, and I-5 on RARγ is better than that of Trifarotene.

### Bioassay example 2: Hepatocyte stability

The metabolic stability of the compounds was tested using human hepatocytes.

### Experimental materials:

| Species | Cell | Product No. | Batch | Source |
|---|---|---|---|---|
| Human | LiverPoor^{™}10-Donation | X008000 | RHZ | BioIVT |

### Experimental steps:

1. The compounds and hepatocytes were incubated for 240 minutes in the following system.

| | |
|---|---|
| Compound concentration | 10 µM |
| Hepatocyte concentration | 1.0×10⁶ cells/mL |

| | |
|---|---|
| Incubation system | WEM+GlutaMAX |
| Time | 0, 240 minutes, 37°C |
| Volume | 200 µL |

2. The culture was inactivated with 2 volumes of acetonitrile (0.1% FA) and then centrifuged for 15 minutes at 16,000 g.
3. The supernatant was analyzed by LC-MS/MS for prototype compounds and metabolites.

### Results:

Results of hepatocyte metabolism for compound I-1:

| Peak | Retention time (minutes) | Molecular weight (m/z) | Molecular weight shift | Normalized area (240 minutes) |
|---|---|---|---|---|
| 1 | 3.60 | 329.16 | -147.06 | 4.84% |
| 2 | 4.19 | 492.24 | 16.02 | 8.84% |
| 3 | 5.03 | 652.27 | 176.05 | 11.60% |
| 4 | 7.34 | 476.24 | 0.02 | 68.15% |
| 5 | 7.76 | 474.23 | -1.99 | 6.45% |
| 6 | 9.72 | 476.22 | 0.00 | 0.12% |

Results of hepatocyte metabolism for Trifarotene:

| Peak | Retention time (minutes) | Molecular weight (m/z) | Molecular weight shift | Normalized area (240 minutes) |
|---|---|---|---|---|
| 1 | 4.18 | 492.24 | 31.99 | 9.35% |
| 2 | 5.03 | 652.27 | 192.03 | 9.98% |
| 3 | 7.32 | 476.24 | 16.00 | 54.29% |
| 4 | 7.76 | 492.24 | 31.99 | 9.10% |
| 5 | 9.50 | 460.25 | 0.00 | 13.57% |

The results show that compound I-1 is metabolized faster in human hepatocytes, with less than 1% of the prototype compound remaining after 240 minutes of incubation in hepatocytes, while Trifarotene still has 13.57% of the prototype compound after 240 minutes of incubation, indicating that compound I-1 has a lower systemic exposure when it enters the blood circulatory system and is expected to have a better safety profile.

## Claims

1. A compound of formula (I): 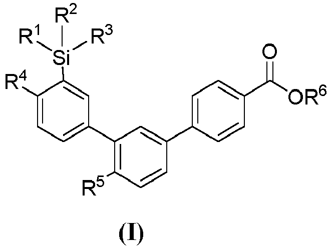
or a tautomer thereof, a stereoisomer thereof, an isotope derivative thereof, or a pharmaceutically acceptable salt thereof; wherein
R¹, R², and R³ are each independently C₁₋₆ alkyl; or, any two of R¹, R², and R³, together with the Si atom to which they are attached, form a 4- to 12-membered heterocycloalkyl ring, wherein the 4- to 12-membered heterocycloalkyl ring comprises 1, 2, 3, or 4 heteroatoms independently selected from Si, N, O, and S;
R⁴ is C₁₋₆ alkyl, -NR^{4a}R^{4b}, or -SiR^{4c}R^{4d}R^{4e}; or, R⁴ and any one of R¹, R², and R³, together with the atom to which they are attached, render 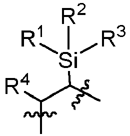 form 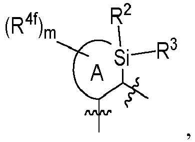 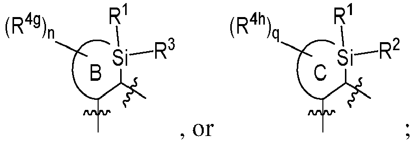
R^{4a} and R^{4b} are each independently C₁₋₆ alkyl; or, R^{4a} and R^{4b}, together with the N atom to which they are attached, form a 4- to 6-membered heterocycloalkyl ring, and the 4- to 6-membered heterocycloalkyl ring comprises 1, 2, or 3 N atoms; R^{4c}, R^{4d}, and R^{4e} are each independently C₁₋₆ alkyl;
ring A, ring B, and ring C are each independently a 4- to 12-membered heterocyclic ring, and the 4- to 12-membered heterocyclic ring comprises 1, 2, 3, or 4 heteroatoms independently selected from Si, N, O, and S; R^{4f}, R^{4g}, or R^{4h} are each independently C₁₋₆ alkyl; m, n, and q are each independently 0, 1, 2, or 3;
R⁵ is C₁₋₆ alkoxy or C₁₋₆ alkoxy substituted by one or more than one R^{5a};
each R^{5a} is independently halogen or hydroxyl;
R⁶ is H or C₁₋₆ alkyl.

2. The compound of formula (I) according to claim 1, wherein
in the definition of R¹, R², and R³, each C₁₋₆ alkyl is independently C₁₋₄ alkyl, for example, methyl, ethyl, n-propyl, isopropyl, n-butyl, isobutyl, or tert-butyl, for another example, methyl;
and/or, in the definition of R¹, R², and R³, when any two of R¹, R², and R³, together with the Si atom to which they are attached, form a 4- to 12-membered heterocycloalkyl ring, each 4- to 12-membered heterocycloalkyl ring is independently a 5- to 10-membered heterocycloalkyl ring, such as a 5- to 6-membered heterocycloalkyl ring;
and/or, in the definition of R¹, R², and R³, when any two of R¹, R², and R³, together with the Si atom to which they are attached, form a 4- to 12-membered heterocycloalkyl ring, each 4- to 12-membered heterocycloalkyl ring independently comprises 2 Si atoms and 0, 1, or 2 heteroatoms independently selected from N, O, and S, such as 2 Si atoms;
and/or, in the definition of R⁴, each C₁₋₆ alkyl is independently C₁₋₄ alkyl, such as methyl, ethyl, *n*-propyl, isopropyl, *n*-butyl, isobutyl, or *tert*-butyl;
and/or, in the definition of R^{4a} and R^{4b}, each C₁₋₆ alkyl is independently C₁₋₄ alkyl, such as methyl, ethyl, *n*-propyl, isopropyl, *n*-butyl, isobutyl, or *tert*-butyl, such as ethyl;
and/or, in the definition of R^{4a} and R^{4b}, when R^{4a} and R^{4b}, together with the N atom to which they are attached, form a 4- to 6-membered heterocycloalkyl ring, the 4- to 6-membered heterocycloalkyl ring is a 5-membered heterocycloalkyl ring;
and/or, in the definition of R^{4a} and R^{4b}, when R^{4a} and R^{4b}, together with the N atom to which they are attached, form a 4- to 6-membered heterocycloalkyl ring, the 4- to 6-membered heterocycloalkyl ring comprises 1 N atom; for example, the 4- to 6-membered heterocycloalkyl ring is 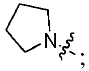
and/or, in the definition of R^{4c}, R^{4d}, and R^{4e}, each C₁₋₆ alkyl is independently C₁₋₄ alkyl, such as methyl, ethyl, *n*-propyl, isopropyl, *n*-butyl, isobutyl, or *tert*-butyl;
and/or, in the definition of R^{4f}, R^{4g}, and R^{4h}, each C₁₋₆ alkyl is independently C₁₋₄ alkyl, for example, methyl, ethyl, *n*-propyl, isopropyl, *n*-butyl, isobutyl, or *tert*-butyl, for another example, methyl;
and/or, in the definition of ring A, ring B, and ring C, each 4- to 12-membered heterocyclic ring is independently a 5- to 10-membered heterocycloalkyl ring, for example, a 5- to 6-membered heterocyclic ring, for another example, a 6-membered heterocyclic ring;
and/or, in the definition of ring A, ring B, and ring C, each 4- to 12-membered heterocyclic ring independently comprises 2 Si atoms and 0, 1, or 2 heteroatoms independently selected from N, O, and S, such as 2 Si atoms;
and/or, in the definition of R⁵, the C₁₋₆ alkoxy is C₁₋₄ alkoxy, for example, methoxy, ethoxy, *n*-propoxy, isopropoxy, *n*-butoxy, isobutoxy, or *tert*-butoxy, for another example, ethoxy, *n-*propoxy, or *n*-butoxy;
and/or, in the definition of R^{5a}, the halogen is fluorine, chlorine, bromine, or iodine;
and/or, in the definition of R⁶, the C₁₋₆ alkyl is C₁₋₄ alkyl, such as methyl, ethyl, *n*-propyl, isopropyl, *n*-butyl, isobutyl, or *tert-butyl.*

3. The compound of formula (I) according to claim 1 or 2, wherein
R¹, R², and R³ are each independently C₁₋₆ alkyl, such as methyl;
and/or, any two of R¹, R², and R³ are each independently C₁₋₆ alkyl, such as methyl;
and/or, R⁴ is -NR^{4a}R^{4b}, such as 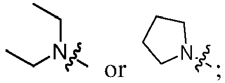
and/or, R^{4a} is C₁₋₆ alkyl, such as ethyl;
and/or, R^{4b} is C₁₋₆ alkyl, such as ethyl;
and/or, R^{4a} and R^{4b} are C₁₋₆ alkyl, such as ethyl;
and/or, each R^{4f} is independently C₁₋₆ alkyl, such as methyl;
and/or, each R^{4g} is independently C₁₋₆ alkyl, such as methyl;
and/or, each R^{4h} is independently C₁₋₆ alkyl, such as methyl;
and/or, m is 2;
and/or, n is 2;
and/or, q is 2;
and/or, 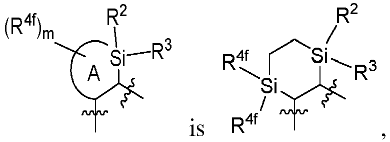 such as 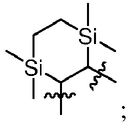
and/or, 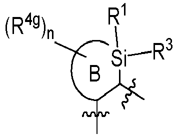 is 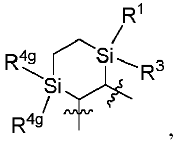 such as 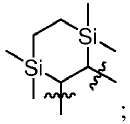
and/or, 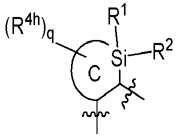 is 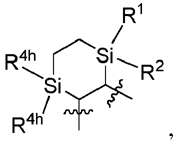 such as 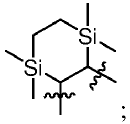
and/or, R⁵ is C₁₋₆ alkoxy substituted by one or more than one R^{5a}, for example, C₁₋₆ alkoxy substituted by one R^{5a}, for another example, -OCH₂CH₂OH, -OCH₂CH₂CH₂OH, or - OCH₂CH₂CH₂CH₂OH;
and/or, R^{5a} is hydroxyl;
and/or, R⁶ is H.

4. The compound of formula (I) according to any one of claims 1 to 3, wherein
R¹, R², and R³ are each independently C₁₋₆ alkyl;
R⁴ is -NR^{4a}R^{4b};
R^{4a} and R^{4b} are each independently C₁₋₆ alkyl; or, R^{4a} and R^{4b}, together with the N atom to which they are attached, form a 4- to 6-membered heterocycloalkyl ring, and the 4- to 6-membered heterocycloalkyl ring comprises 1, 2 or 3 N atoms;
R⁵ is C₁₋₆ alkoxy substituted by one or more than one R^{5a};
each R^{5a} is independently halogen or hydroxyl;
R⁶ is H or C₁₋₆ alkyl.

5. The compound of formula (I) according to any one of claims 1 to 3, wherein
two of R¹, R², and R³ are each independently C₁₋₆ alkyl;
R⁴ and any one of R¹, R², and R³, together with the atom to which they are attached, render 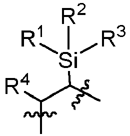 form 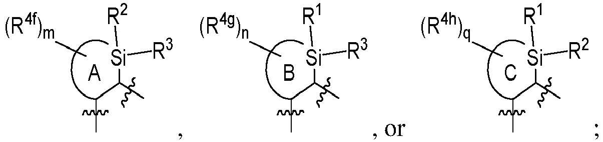
wherein ring A, ring B, and ring C are each independently a 4- to 12-membered heterocyclic ring, and the 4- to 12-membered heterocyclic ring comprises 1, 2, 3, or 4 heteroatoms independently selected from Si, N, O, and S;
R^{4f}, R^{4g}, and R^{4h} are each independently C₁₋₆ alkyl;
m, n, and q are each independently 0, 1, 2, or 3;
R⁵ is C₁₋₆ alkoxy substituted by one or more than one R^{5a};
each R^{5a} is independently halogen or hydroxyl;
R⁶ is H or C₁₋₆ alkyl.

6. The compound of formula (I) according to any one of claims 1 to 5, wherein the compound of formula (I) has a structure of formula (I-a), (I-b), (I-c), or (I-d): 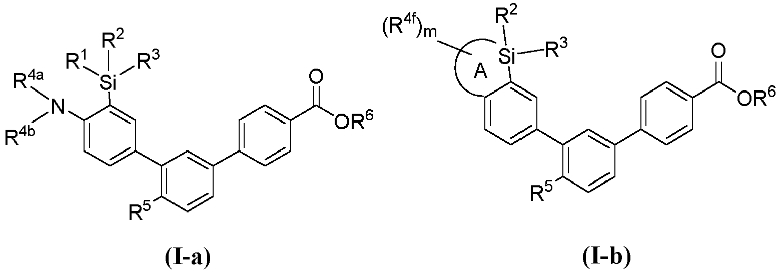 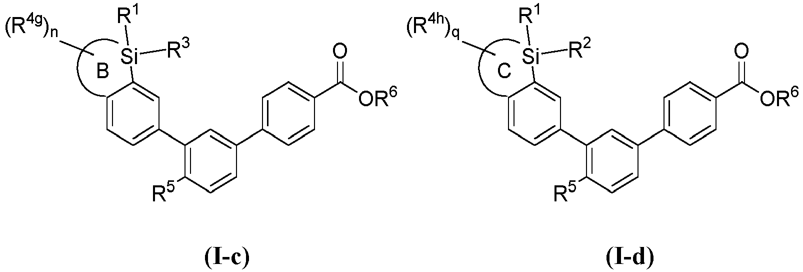 wherein ring A, ring B, ring C, m, n, q, R^{4a}, R^{4b}, R^{4f}, R^{4g}, R^{4h}, R¹, R², R³, R⁵, and R⁶ are as defined in any one of claims 1 to 5.

7. The compound of formula (I) according to any one of claims 1 to 6, wherein the compound of formula (I) has a structure of formula (I-b-1), (I-c-1), or (I-d-1): 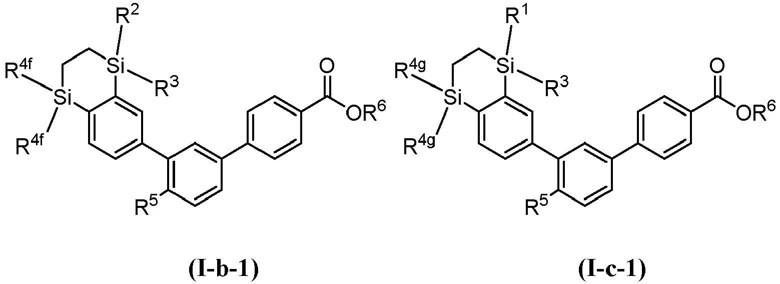 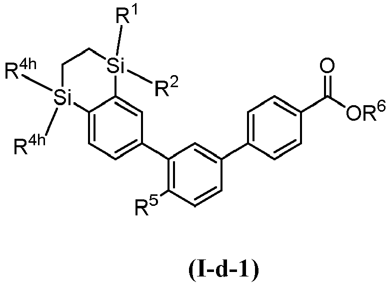 wherein R^{4f}, R^{4g}, R^{4h}, R¹, R², R³, R⁵, and R⁶ are as defined in any one of claims 1 to 6.

8. The compound of formula (I) according to any one of claims 1 to 7, wherein the compound of formula (I) is:

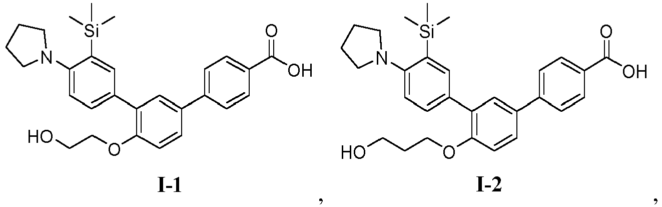

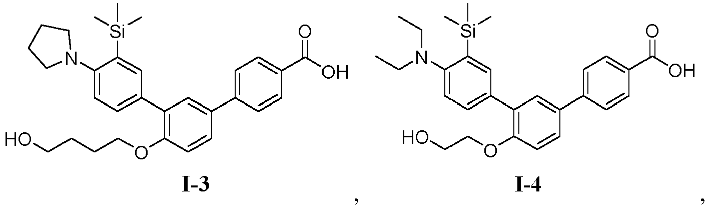

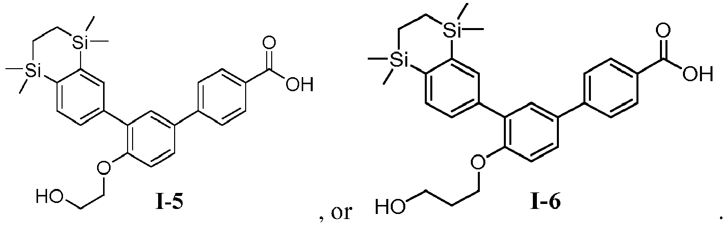

9. A preparation method for the compound of formula (I) according to any one of claims 1 to 8, wherein the preparation method is any one of the following:
method (A) comprises the following steps: in a solvent, reacting a compound of formula (II-a) as shown below in the presence of a base to obtain the compound of formula (I); wherein R⁵ contains a hydroxyl group, R^{5a} is a group in which the hydroxyl group in R⁵ is protected by a hydroxyl protecting group, R⁶ is H, and 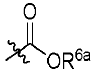 is a group in which 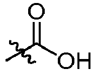 is protected by a carboxyl protecting group; 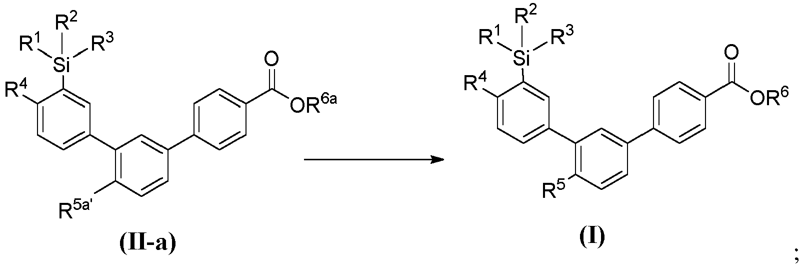
method (B) comprises the following steps: in a solvent, reacting a compound of formula (II-b) as shown below in the presence of a base to obtain the compound of formula (I); wherein R⁵ does not contain a hydroxyl group, R⁶ is H, R^{5b} and R⁵ are the same, and 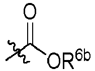 is a group in which 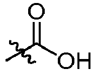 is protected by a carboxyl protecting group; 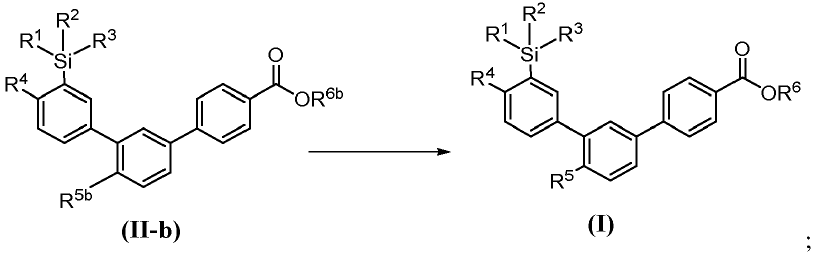
method (C) comprises the following steps: in a solvent, reacting a compound of formula (II-c) as shown below in the presence of a base to obtain the compound of formula (I); wherein R⁵ contains a hydroxyl group, R⁶ is not H, R^{5c} is a group in which the hydroxyl group in R⁵ is protected by a hydroxyl protecting group, and R^{6c} and R⁶ are the same; 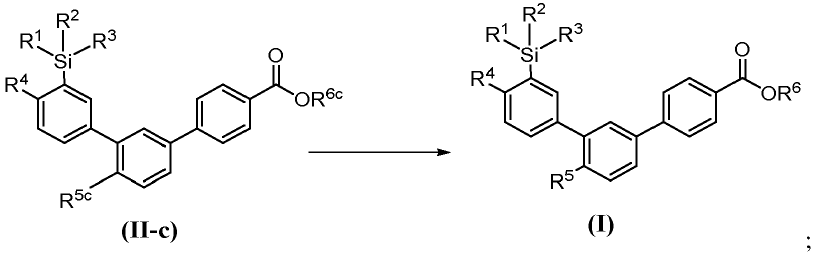
R¹, R², R³, and R⁴ are as defined in any one of claims 1 to 5.

10. A preparation method for a compound of formula (II-a), (II-b), or (II-c), wherein
the preparation method for the compound of formula (II-a) comprises the following steps: in a solvent, performing a coupling reaction as shown below between a compound of formula (III-a) and a compound of formula (IV) to obtain the compound of formula (II-a); 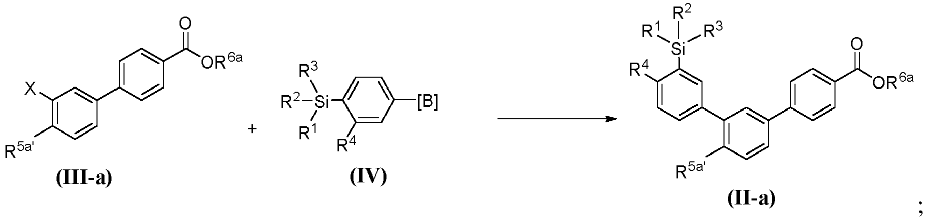
the preparation method for the compound of formula (II-b) comprises the following steps: in a solvent, performing a coupling reaction as shown below between a compound of formula (III-b) and the compound of formula (IV) to obtain the compound of formula (II-b); 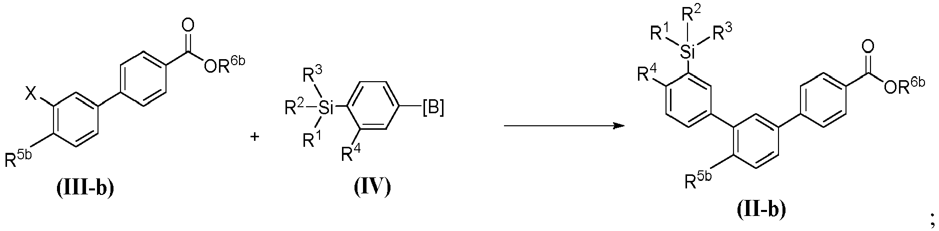
the preparation method for the compound of formula (II-c) comprises the following steps: in a solvent, performing a coupling reaction as shown below between a compound of formula (III-c) and the compound of formula (IV) to obtain the compound of formula (II-c); 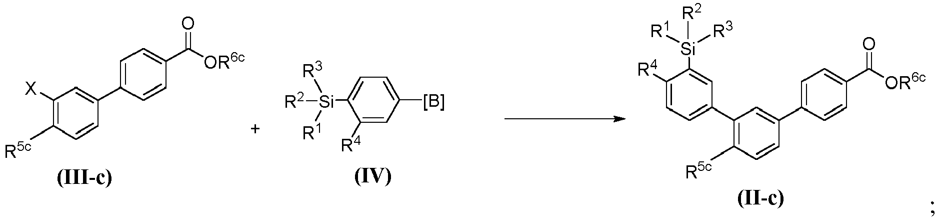
wherein [B] is boric acid or borate ester, such as 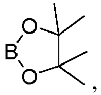 R¹, R², R³, and R⁴ are as defined in any one of claims 1 to 5; R^{5a'}, R^{5b}, R^{5c}, R^{6a}, R^{6b}, and R^{6c} are as defined in claim 9.

11. A preparation method for a compound of formula (IV), wherein
when R⁴ in the compound of formula (IV) is C₁₋₆ alkyl, -NR^{4a}R^{4b}, or -SiR^{4c}R^{4d}R^{4e}, and [B] is 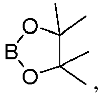 the preparation method comprises the following steps: in a solvent, performing a coupling reaction as shown below between a compound of formula (V) and bis(pinacolato)diboron to obtain the compound of formula (IV); 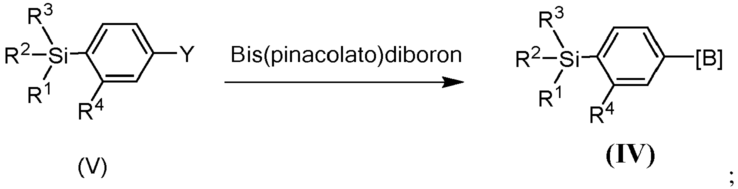
wherein Y is Br or I, such as Br;
when the compound of formula (IV) is a compound of formula (IV-a-1), (IV-b-1), or (IV-c-1), the preparation method comprises the following steps: in a solvent, performing a cyclization reaction as shown below between a compound of formula (VI-a), (VI-b), or (VI-c) and 2-ethynyl-4,4,5,5-tetramethyl-[1,3,2]dioxaborolane to obtain the compound of formula (IV-a-1), (IV-b-1), or (IV-c-1), respectively; 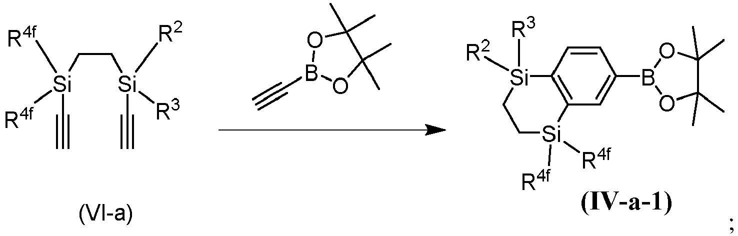 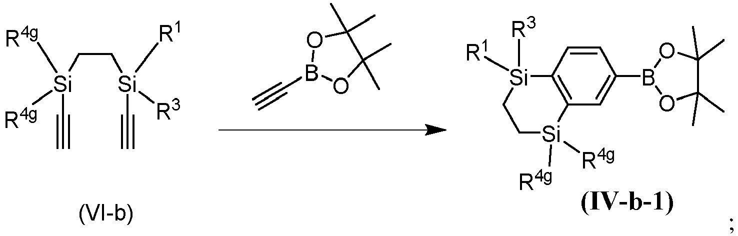 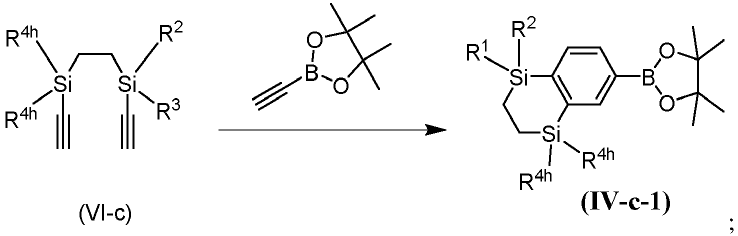
wherein R¹, R², R³, R^{4f}, R^{4g}, and R^{4h} are as defined in any one of claims 1 to 5.

12. A compound of formula (V), (IV), (II-a), (II-b), or (II-c): 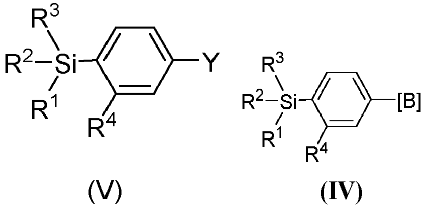 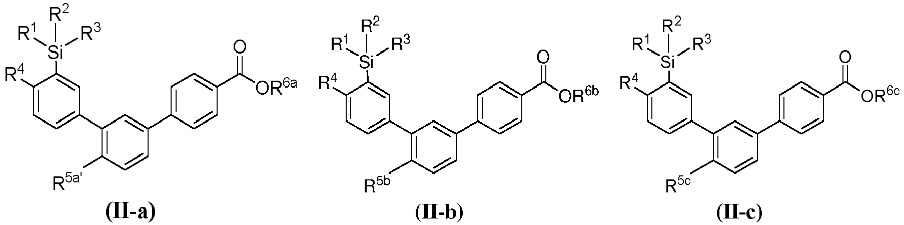 wherein R¹, R², R³, and R⁴ are as defined in any one of claims 1 to 5, R^{5a'}, R^{5b}, R^{5c}, R^{6a}, R^{6b}, and R^{6c} are as defined in claim 9, [B] is as defined in claim 10, and Y is as defined in claim 11.

13. The compound according to claim 12, wherein the compound of formula (V), (IV), or (II-a) is:

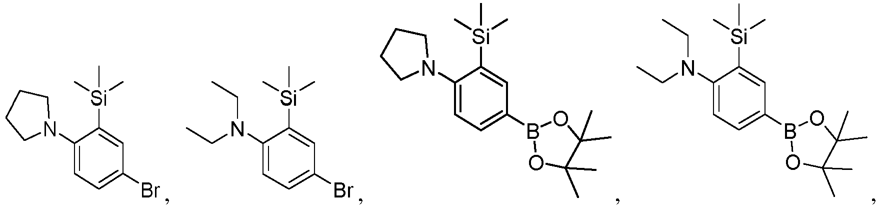

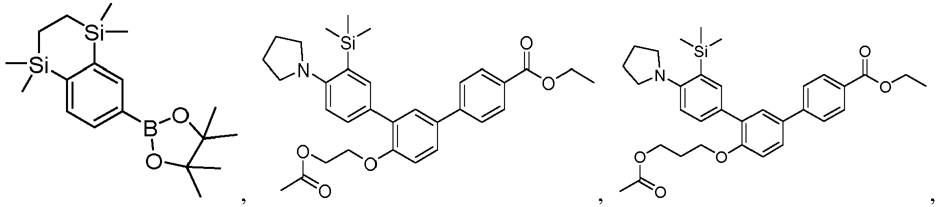

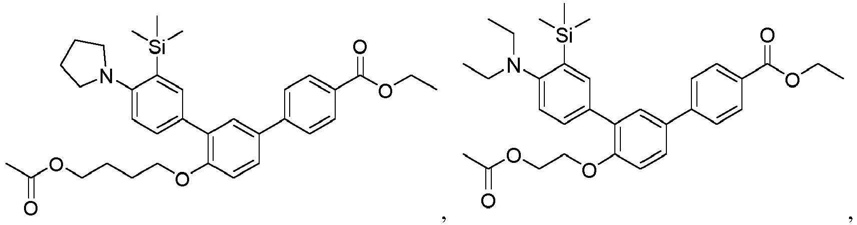

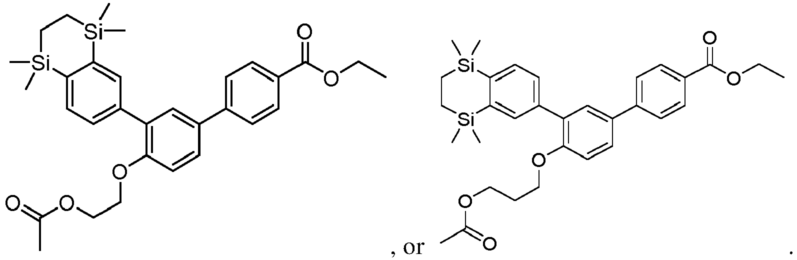

14. A pharmaceutical composition comprising (i) the compound of formula (I), the tautomer thereof, the stereoisomer thereof, the isotope derivative thereof, or the pharmaceutically acceptable salt thereof according to any one of claims 1 to 8, and (ii) a pharmaceutically acceptable carrier.

15. A use of the compound of formula (I), the tautomer thereof, the stereoisomer thereof, the isotope derivative thereof, or the pharmaceutically acceptable salt thereof according to any one of claims 1 to 8, or the pharmaceutical composition according to claim 14 in the manufacture of a medicament for the treatment or prevention of a disease associated with retinoic acid receptor;
wherein the disease associated with retinoic acid receptor is preferably a disease associated with retinoic acid receptor γ, such as acne, pimples, psoriasis, ichthyosis, keratosis, hyperpigmentation, and dry eye syndrome, wherein the acne is preferably common acne, rosacea, nodulocystic acne, acne conglobata, and secondary acne caused by sun exposure or medication therapy.
